# EUROPEAN PATENT APPLICATION

(11) **EP 3 944 861 A1**
(43) Date of publication of application: **02.02.2022**
(21) Application number: 20188751.0
(22) Date of filing: 30.07.2020
(51) Int. Cl.: A61K 31/7088, C12N 15/113

(54) **LONG NON-CODING RNA AS THERAPEUTIC TARGET IN CARDIAC DISORDERS AND CARDIAC REGENERATION**

(71) Applicant: Medizinische Hochschule Hannover, 30625 Hannover (DE)
(72) Inventor: THUM, Thomas, 30627 Hannover (DE); CHENG-KAI, Huang, 30625 Hannover (DE); BÄR, Christian, 30159 Hannover (DE)
(74) Representative: Weiss, Wolfgang

(57) **Abstract**

The present invention relates to a long non-coding RNA as a therapeutic target in cardiac disorders.

SEQUENCE LISTING

<110>  Medizinische Hochschule Hannover

<120>  Long non-coding RNA as therapeutic target in cardiac disorders
       and cardiac regeneration

<130>  70896P EP

<160>  57

<170>  PatentIn version 3.5

<210>  1
<211>  623
<212>  DNA
<213>  human


<220>
<221>  exon
<222>  (1)..(334)

<220>
<221>  exon
<222>  (335)..(465)

<220>
<221>  exon
<222>  (466)..(623)

<400>  1
gcctgccgac ccgggtccgg gggcccagtt ggcgcccgga ccagctctcc ggtctgcgtc      60

ctgtagatgg gaaaactgag gcctgagagg gcgcgagggc ggggacgagg ccgaggactc     120

gggttcgaat ctcgaggacg ccagtagccc gctctgaggc cgcgtggggc tggtggagga     180

gccaccggtc gggcccccaa gaccggctga cgtgggcctc gcctcgggcg actcccgccg     240

ggctagtggc tggccgtgtg gtgaccttgg gcgagttact tcgcacagcc acccaggggc     300

ctcttctcca acacgatagc actgcacccg gagggccttc agacctgagc tgaaatctac     360

accaccggct ctctgactat caggctttgg actatacttt gggctttcct gggtctctta     420

cctggcagac agcagatttt ggaacttttc agcctccata atcatttccc agaagaacca     480

caaagcataa atttccccaa aaggggaatt ttgttgatgg ctttagtcta ttccctccta     540

aaagaccagc tacaaccaaa tgaagtgaac ataacctcaa gggtgtattg tcttcataat     600

aaaagatgaa gcttagaact gga                                             623


<210>  2
<211>  709
<212>  DNA
<213>  human

<400>  2
ccggctgacg tgggcctcgc ctcgggcgac tcccgccggg ctagtggctg gccgtgtggt      60

gaccttgggc gagttacttc gcacagccac ccaggggcct cttctccaac acgatagcac 120

tgcacccgga ggataaagaa ccactgagga tatgagttat tcttcctaca gttgtacagc 180

ttgtgaacag tagagctggg gtccgagcca ggtctctgac tctgcagcct acccttgtcc 240

tgcttgaaca cactgaggaa gacaggaata acaggacatg ggtcagaagg cttggcttct 300

gctccagctt tgaagttgaa ggattaggtg acagctaaca cttgatagta ttttatggtt 360

tcaaagtgat aaccatgtaa tattttaatt gattctcagc ccattttgta gatgaggaaa 420

ctgaggctca cagatctgat gacttcctaa ttgtggcaaa ctaccagcta cacagtagct 480

gggctggctt cgaaccaggt cttttgaccc cagagtctgt gcagctcaca aataaagctg 540

attctttgac aagttaagcc tcttctcttg gattcatttt tccagtctgt gaaaatgagg 600

gattggatta gattagtagg caatcactag ttcccaggcc cttttattta aagaaaaaaa 660

ggagaaccca tagagtctgg caacttaaac ttttgctaag tatgaacac 709

<210> 3
<211> 514
<212> DNA
<213> human

<400> 3
ctgactatca ggctttggac tatactttgg gctttcctgg gtctcttacc tggcagacag 60

cagattttgg aacttttcag cctccataat catcaactct gtgagggatt gttatctcca 120

ttttatggat gaggaagctg actctgagag atgactttcc aaattcatcc aactggaaga 180

aaaaggaagt ctctggggac ttgaatccag aactgtccag ctccaagtcc agggctcact 240

ctattaagcc actccatgag gaatgagtcc ctaaggaggc cacagaaggc cctaagccct 300

cttttgttct ctggaagaga gtgccatttc ttggcccttc ccaaggcctt gacaactgaa 360

gttcccagaa gaaccacaaa gcataaattt ccccaaaagg ggaattttgt tgatggcttt 420

agtctattcc ctcctaaaag accagctaca accaaatgaa gtgaacataa cctcaagggt 480

gtattgtctt cataataaaa gatgaagctt agaa 514

<210> 4
<211> 1066
<212> DNA
<213> human

<400> 4
accggtcggg cccccaagac cggctgacgt gggcctcgcc tcgggcgact cccgccgggc 60

tagtggctgg ccgtgtggtg accttgggcg agttacttcg cacagccacc caggggcctc 120

ttctccaaca cgatagcact gcacccggag ggccttcaga cctgagctga aatctacacc 180

accggctctc tgactatcag gctttggact atactttggg ctttcctggg tctcttacct 240

ggcagacagc agattttgga acttttcagc ctccataatc atgcatggaa ttcaaatcgc 300

ggagtgacag gctgtgtgtg gggctgattg tctgggtcag agacatcaca actggctggt 360

gaggagagct ttctgacaac aggagctgtc caacaaggag tgggctgctg tcaccgagga 420

gctgaagcgg ccgacggctg accttccact gggcactagg cagggcctgg atggagggag 480

tcttaaagta ggagggagaa gccaaggaaa tagctctcta gaagcatttt gtacactgaa 540

aagtgtcatt gaaacgctgt cacagttcta agaggagaca ttgctcctct gaaaaccagc 600

ctcactccct aagctgatgt atttccagaa gcagggcctg agctgccaca tgcaggccct 660

gaggacttga ggcacatcgt tctttttttg ggtctcatct gtgaatcagc cactcaaacc 720

tagtggctct cagcctgcaa gttcattcat gcaatgcagg gcttctccac tgctccccca 780

aacctcctca ggccacccct gcgtgtgcac ccaccgatat ggcctgttct gaacaggtga 840

ccctcagctg ccagcatcac tgcccttcaa aacctgactt atctctcgtg ttttacagtt 900

accatagcag aaagatttat cttcatcttt taccatttaa aaatactaat ggaattactg 960

tttttgtctg tttctgtttt gaagccatac gatttttctc tcactgccac cacatttgtg 1020

atggccttaa aaattattct taaagataaa tataattcta gaaaaa 1066

<210> 5
<211> 2291
<212> DNA
<213> mouse

<220>
<221> exon
<222> (1)..(481)

<220>
<221> exon
<222> (482)..(2291)

<400> 5
aggctaaagg gacccagtag catggagggc ccctttagaa aatctaaggc cacttcaatt 60

gcttccacaa tctattccct gggcacaggg ttccttgtgt caggaaagca gatcaagtag 120

ctacggagag ttcccgagaa cacaaggacc tggcccaaag aacctgatgt tctcctttgg 180

ccagaaaact gtccacccat caacagaggt agagaatcaa tgccagagtg aatccttctt 240

accggaacat gcgatccctt tccatcctgc cttcttgtgc cctcagtttc actcagccct 300

cactgagtgc cagcctctgc ggaactcctg actgagaatc taactccttg tgggcaagga 360

cttggcccaa tctattcaca gctctgtcct cggggcctcg aggagaggct gacaatgaga 420

agaagtgcgg ggcaaatgaa agcatgagct cacactattc ttacaaggac cggagaggca 480

gggcatttcg tgtttgggat gcactaatag cctgacaagg gctcagtgca gccgagcaag 540

gctgagtgtg ttctaaaaga gtcgtctgag gattcagccg caatgaccat acactcagag 600

catagagcaa gatacagacc ctgttggtgg ccaccatgcc agaggaggtg gatttgtctc 660

ctgggtttcc tgtggaaaca gattctgtca aggcttgtgt tctgttcagt cacacaggtg 720

gctttgctta ggatgagaag gaagcctgtg agcatctggg tttgaggatg tggggaggac 780

agggatgagg gcaaggagcc tcgggcagtg ctgtgtctgg ttcccaatag gtgcagggaa 840

ggactgagac ctggcatcac agctagcctg ctggtgggcc agccctggag ggacagacaa 900

aatgcctagg tggacagaaa catctccatc tagtagagca cctgtaaagt cacattggaa 960

ccttctaccg cctgtgtttg gctcgctcct caccaaagaa gccgtggaaa ggaaggtgca 1020

acattggggc ttacatgttg gtgtccatga tggccaaaaa gctgcatgac tctacatcgc 1080

tggagttgag tgtcaagtct gaaacaagca agaaagcata cagcatggct ttccttagag 1140

gcttgcaaga acacacacaa cagcagtggg tccccagcac acccctcact tgccagatga 1200

ggaaatggac ccaggaggca aggatttgcc acatatgtag tgtttccatt tatataagag 1260

gtgtccttcc cgaaaggttc atgtataagg ggcttagtcc ctagctggtg gtggtgctgt 1320

tttgggtggt ggtgcaagct gcaagtacca ttagctggga cctagcagat ggaactaagt 1380

atactctgcc acacccacac cctccttgcc tcgaagggct gacccctctg aaaccataag 1440

cccaaataaa tcaattgttt ctttcaggtg tttggtcaga gtgaccacaa aaaaaatttt 1500

tttttcctaa ctgtgtgttg tgaactaagg aagctaatga tagaagccag acagacccag 1560

accttctcac agccagagcc ctaaccctca agtctctgac tgttcttccc tcctaggtgg 1620

tgctatgtca gccacaacct ccagtcagca ctctccactt tcagagatgc tgtctagagc 1680

tgggcaccca tccccatggc ctctatagtg agcggccatg catgctgaga gcaatggctg 1740

atttacagtg gagtgtgttg taagtatgta atgcattatg ttaattcagg ggcacacatt 1800

tatgtgtgtc cacacatgtg tgcatgcata tatggtcagt gcatctacat gtgtgtgtgc 1860

acatgaaggc cagagactga caatgagtac cctcttcagt ggctctccac cttacacact 1920

gaggcaggct ctctcccttg aaccgagagc tcacatattc acctagtgca gctggctggc 1980

ttgctccagg acgtcctgtc tctgtctccc gaatgctggg cttgccggtg ggctgccaca 2040

cccttgtgac atttacatgg gctctaggga tttgaactcc agtcctcggg cttgcatgga 2100

gagcatttaa ccttgagtca tctttccacc ccccaagatt tggagttgtt acatgagtaa 2160

aacttttaaa atttttattt aacaaataaa ttttatttat tcttttagaa tctcgttaag 2220

gatttttaga tactgatcaa atgctgacat gagattcttt taaataggtt gaataaaata 2280

tttttgagtg g 2291

<210> 6
<211> 117
<212> PRT
<213> artificial sequence

<220>
<223>  predicted amino acid translated from the largest open reading
       frame (ORF) in Foxo6os

<400>  6

Met Pro Gly Leu Ser Pro Ser Leu His Leu Leu Gly Thr Arg His Ser
1               5                   10                  15

Thr Ala Arg Gly Ser Leu Pro Ser Ser Leu Ser Ser Pro His Pro Gln
            20                  25                  30

Thr Gln Met Leu Thr Gly Phe Leu Leu Ile Leu Ser Lys Ala Thr Cys
        35                  40                  45

Val Thr Glu Gln Asn Thr Ser Leu Asp Arg Ile Cys Phe His Arg Lys
    50                  55                  60

Pro Arg Arg Gln Ile His Leu Leu Trp His Gly Gly His Gln Gln Gly
65                  70                  75                  80

Leu Tyr Leu Ala Leu Cys Ser Glu Cys Met Val Ile Ala Ala Glu Ser
                85                  90                  95

Ser Asp Asp Ser Phe Arg Thr His Ser Ala Leu Leu Gly Cys Thr Glu
            100                 105                 110

Pro Leu Ser Gly Tyr
        115


<210>  7
<211>  12
<212>  DNA
<213>  artificial sequence

<220>
<223>  Sequencing chromatograms showed the mutant junction site of
       CRISPRed HL-1 cells gDNA.

<400>  7
tcctgaccca ag                                                          12


<210>  8
<211>  6
<212>  DNA
<213>  artificial sequence

<220>
<223>  Predicted sequence of mouse Foxo6os promoter

<400>  8
tgggga                                                                  6

<210>  9
<211>  6
<212>  DNA
<213>  artificial sequence

<220>
<223>  Predicted sequence of mouse Foxo6os promoter

<400>  9
tgggga                                                                 6


<210>  10
<211>  6
<212>  DNA
<213>  artificial sequence

<220>
<223>  Predicted sequence of mouse Foxo6os promoter

<400>  10
tgggga                                                                 6


<210>  11
<211>  10
<212>  DNA
<213>  artificial sequence

<220>
<223>  Predicted sequence of mouse Foxo6os promoter

<400>  11
tgcacgtgta                                                            10


<210>  12
<211>  9
<212>  DNA
<213>  artificial sequence

<220>
<223>  Predicted sequence of mouse Foxo6os promoter

<400>  12
atgcccagc                                                              9


<210>  13
<211>  12
<212>  DNA
<213>  artificial sequence

<220>
<223>  Predicted sequence of mouse Foxo6os promoter

<400>  13
gtttgtttgt tt                                                         12


<210>  14
<211>  6

<212>  DNA
<213>  artificial sequence

<220>
<223>  Predicted sequence of mouse Foxo6os promoter

<400>  14
cgggga                                                                  6


<210>  15
<211>  13
<212>  DNA
<213>  artificial sequence

<220>
<223>  Predicted sequence of mouse Foxo6os promoter

<400>  15
aaggtgtgaa agc                                                         13


<210>  16
<211>  9
<212>  DNA
<213>  artificial sequence

<220>
<223>  Predicted sequence of mouse Foxo6os promoter

<400>  16
ggtgccagc                                                               9


<210>  17
<211>  9
<212>  DNA
<213>  artificial sequence

<220>
<223>  Predicted sequence of mouse Foxo6os promoter

<400>  17
agtgccagc                                                               9


<210>  18
<211>  6
<212>  DNA
<213>  artificial sequence

<220>
<223>  Predicted sequence of mouse Foxo6os promoter

<400>  18
ggggga                                                                  6


<210>  19
<211>  6
<212>  DNA
<213>  artificial sequence

<220>
<223>  Predicted sequence of mouse Foxo6os promoter

<400>  19
   ggggga                                                                   6


<210>  20
<211>  6
<212>  DNA
<213>  artificial sequence

<220>
<223>  Predicted sequence of mouse Foxo6os promoter

<400>  20
   ggggga                                                                   6


<210>  21
<211>  7
<212>  DNA
<213>  artificial sequence

<220>
<223>  Predicted sequence of mouse Foxo6os promoter

<400>  21
cacgtgt                                                                 7


<210>  22
<211>  11
<212>  DNA
<213>  artificial sequence

<220>
<223>  Predicted sequence of mouse Foxo6os promoter

<400>  22
tacacgtgca c                                                           11


<210>  23
<211>  12
<212>  DNA
<213>  artificial sequence

<220>
<223>  Predicted sequence of mouse Foxo6os promoter

<400>  23
ttttatttgt tt                                                          12


<210>  24
<211>  11
<212>  DNA
<213>  artificial sequence

<220>
<223>  Predicted sequence of mouse Foxo6os promoter

<400>  24
gtcacctgca c                                                            11


<210>  25
<211>  10
<212>  PRT
<213>  artificial sequence

<220>
<223>  Predicted sequence of mouse Foxo6os promoter

<400>  25

Cys Cys Cys Gly Gly Ala Ala Gly Thr Gly
1               5                   10


<210>  26
<211>  10
<212>  DNA
<213>  artificial sequence

<220>
<223>  Predicted sequence of mouse Foxo6os promoter

<400>  26
cacccggaag                                                              10


<210>  27
<211>  12
<212>  DNA
<213>  artificial sequence

<220>
<223>  Predicted sequence of mouse Foxo6os promoter

<400>  27
atttgtttgt tt                                                           12


<210>  28
<211>  9
<212>  DNA
<213>  artificial sequence

<220>
<223>  Predicted sequence of mouse Foxo6os promoter

<400>  28
cctgccagg                                                                9


<210>  29
<211>  6
<212>  DNA
<213>  artificial sequence

<220>
<223>  Predicted sequence of mouse Foxo6os promoter

<400>  29

agggga                                                                 6


<210>  30
<211>  8
<212>  DNA
<213>  artificial sequence

<220>
<223>  Predicted sequence of mouse Foxo6os promoter

<400>  30
tgtctggg                                                               8


<210>  31
<211>  9
<212>  DNA
<213>  artificial sequence

<220>
<223>  Predicted sequence of mouse Foxo6os promoter

<400>  31
gtgccagct                                                              9


<210>  32
<211>  9
<212>  DNA
<213>  artificial sequence

<220>
<223>  Predicted sequence of mouse Foxo6os promoter

<400>  32
gcagccaac                                                              9


<210>  33
<211>  11
<212>  DNA
<213>  artificial sequence

<220>
<223>  Predicted sequence of human AC093151.3 promoter

<400>  33
gccccgcccc c                                                          11


<210>  34
<211>  11
<212>  DNA
<213>  artificial sequence

<220>
<223>  Predicted sequence of human AC093151.3 promoter

<400>  34
gccccgcccc c                                                          11

<210>  35
<211>  11
<212>  DNA
<213>  artificial sequence

<220>
<223>  Predicted sequence of human AC093151.3 promoter

<400>  35
acatggcggc g                                                            11


<210>  36
<211>  10
<212>  DNA
<213>  artificial sequence

<220>
<223>  Predicted sequence of human AC093151.3 promoter

<400>  36
ggcgcgcgcc                                                              10


<210>  37
<211>  10
<212>  DNA
<213>  artificial sequence

<220>
<223>  Predicted sequence of human AC093151.3 promoter

<400>  37
ggcgcgcgcc                                                              10


<210>  38
<211>  15
<212>  DNA
<213>  artificial sequence

<220>
<223>  Predicted sequence of human AC093151.3 promoter

<400>  38
gccccgcccc ccgcc                                                        15


<210>  39
<211>  10
<212>  DNA
<213>  artificial sequence

<220>
<223>  Predicted sequence of human AC093151.3 promoter

<400>  39
ggggcggggc                                                              10


<210>  40
<211>  10
<212>  DNA

<213>  artificial sequence

<220>
<223>  Predicted sequence of human AC093151.3 promoter

<400>  40
ggggcggggc                                                              10


<210>  41
<211>  6
<212>  DNA
<213>  artificial sequence

<220>
<223>  Predicted sequence of human AC093151.3 promoter

<400>  41
cgcgtg                                                                   6


<210>  42
<211>  11
<212>  DNA
<213>  artificial sequence

<220>
<223>  Predicted sequence of human AC093151.3 promoter

<400>  42
agggcgcgag g                                                            11


<210>  43
<211>  11
<212>  DNA
<213>  artificial sequence

<220>
<223>  Predicted sequence of human AC093151.3 promoter

<400>  43
gccccgcccc c                                                            11


<210>  44
<211>  11
<212>  DNA
<213>  artificial sequence

<220>
<223>  Predicted sequence of human AC093151.3 promoter

<400>  44
gccccgcccc c                                                            11


<210>  45
<211>  11
<212>  DNA
<213>  artificial sequence

<220>

<223>  Predicted sequence of human AC093151.3 promoter

<400>  45
cggcgggaag a                                                            11


<210>  46
<211>  11
<212>  DNA
<213>  artificial sequence

<220>
<223>  Predicted sequence of human AC093151.3 promoter

<400>  46
cccccgcccc g                                                            11


<210>  47
<211>  15
<212>  DNA
<213>  artificial sequence

<220>
<223>  Predicted sequence of human AC093151.3 promoter

<400>  47
gccccgcccc ccgcg                                                        15


<210>  48
<211>  11
<212>  DNA
<213>  artificial sequence

<220>
<223>  Predicted sequence of human AC093151.3 promoter

<400>  48
gccccgcccg c                                                            11


<210>  49
<211>  6
<212>  DNA
<213>  artificial sequence

<220>
<223>  Predicted sequence of human AC093151.3 promoter

<400>  49
cgggga                                                                   6


<210>  50
<211>  10
<212>  DNA
<213>  artificial sequence

<220>
<223>  Predicted sequence of human AC093151.3 promoter

<400>  50

ggcacgagct                                                              10


<210>  51
<211>  10
<212>  DNA
<213>  artificial sequence

<220>
<223>  Predicted sequence of human AC093151.3 promoter

<400>  51
agctcgtgcc                                                              10


<210>  52
<211>  8
<212>  DNA
<213>  artificial sequence

<220>
<223>  Predicted sequence of human AC093151.3 promoter

<400>  52
cagaaggc                                                                8


<210>  53
<211>  7
<212>  DNA
<213>  artificial sequence

<220>
<223>  Predicted sequence of human AC093151.3 promoter

<400>  53
ctgacac                                                                 7


<210>  54
<211>  8
<212>  DNA
<213>  artificial sequence

<220>
<223>  Predicted sequence of human AC093151.3 promoter

<400>  54
tgtctggc                                                                8


<210>  55
<211>  8
<212>  DNA
<213>  artificial sequence

<220>
<223>  Predicted sequence of human AC093151.3 promoter

<400>  55
tgtctggc                                                                8

<210>  56
<211>  11
<212>  DNA
<213>  artificial sequence

<220>
<223>  Predicted sequence of human AC093151.3 promoter

<400>  56
cgcgcgggaa c                                                            11


<210>  57
<211>  11
<212>  DNA
<213>  artificial sequence

<220>
<223>  Predicted sequence of human AC093151.3 promoter

<400>  57
cgcctggggc c                                                            11

## Description

The present invention relates to a long non-coding RNA as a therapeutic target in cardiac disorders.

Heart failure is one of the leading pathological causes of mortality in the world. Myocardial infarction (MI) is the most important cause of heart failure as MI leads to subsequent progressive remodeling of the heart resulting in heart failure with poor prognosis. The currently used therapeutic pharmacologic options for heart failure include angiotensin-modulating agents, β-blockers, diuretics, aldosterone antagonists, a combined neprilysin-inhibitor with angiotensin-II-receptor blocker, vasodilators, or inotropic agents. Although several clinical studies have shown significant decreases in heart failure-induced mortality rates for all these agents, the 5-year mortality rate remains unacceptably at almost 50%. Thus, there is a great need to develop novel and more efficient therapeutic approaches for heart failure.

Pathological hypertrophic growth of cardiomyocytes can lead to the development of cardiac remodeling, heart failure and sudden cardiac death. Hypertrophic growth of cardiomyocytes is a response to increased cardiac wall stress caused by cardiac volume and/or pressure overload. Initially, cardiac hypertrophy is a compensatory mechanism aiming to decrease wall stress and to increase cardiac output. However, prolonged cardiac hypertrophy progresses to contractile dysfunction, cardiac decompensation and finally heart failure (Hill and Olson, 2008; Barry and Townsend, 2010). The transition from physiological to pathological hypertrophy can occur depending on many factors including myocyte loss through apoptosis or necrosis, alterations in autophagy, defects in contractile response, dysregulated calcium homeostasis, desensitization of adrenergic receptors, or cardiac fibrosis (Hill and Olson, 2008; Barry and Townsend, 2010). Hypertrophic signaling is largely mediated by the insulin signaling pathway (DeBosch and Muslin, 2008; Barry and Townsend, 2010). Both insulin and insulin-like growth factor-1 (IGF-1) activate pro-hypertrophic pathways in cardiomyocytes via the IGF-1 receptor, which activates the phosphoinositine-3-kinase (PI3K) (McMullen et al., 2004). PI3K activity leads to the activation of the serine/threonine kinase Akt via its phosphorylation and active Akt phosphorylates anti-hypertrophic FoxO transcription factors leading to their de-stabilization and prevention of nuclear localization (Datta et al., 1999; Skurk et al., 2005; Ronnebaum and Patterson, 2010). In contrast, acetylation of FoxO factors by sirtuin-1 (Sirt-1) leads to their stabilization and nuclear translocation (Frescas et al., 2005). Stabilized FoxO transcription factors are localized in the nucleus in order to regulate the expression of anti-hypertrophic genes. The anti-hypertrophic functions of FoxO proteins are largely mediated through suppression of the pro-hypertrophic calcineurin signaling pathway via the expression of anti-hypertrophic gene targets of FoxO factors, such as atrogin-1 (Ni et al., 2006; Ronnebaum and Patterson, 2010; Glas, 2010). Moreover, FoxO transcription factors also induce apoptosis and regulate autophagy in cardiomyocytes (Ronnebaum and Patterson, 2010).

The present inventors have identified a long non-coding RNA (IncRNA) which plays an important role in both heart development and heart disease. The respective murine IncRNA Foxo6os is specifically expressed in cardiomyocytes and highly up-regulated during heart development. In contrast thereto, Foxo6os is down-regulated in three different heart disease models. Further, the inventors have identified a human locus-conserved transcript AC093151.3, which is up-regulated during the differentiation of human-induced pluripotent stem cells into cardiomyocytes and is down-regulated in hypertrophic stimuli and hypoxic conditions, thus, paralleling the in vivo observations in mice. Myeloid zinc finger 1 (Mzf1), a known transcription factor that regulates Nkx2.5 expression and heart development (Doppler et al. 2014), was predicted to regulate Foxo6os transcription.

Further, the present inventors have identified several Foxo6os-binding proteins, particularly the proteins CIRBP and RBM3, both of which belong to a highly conserved cold shock proteins family, characterized by an N-terminal RNA recognition motif (RRM) and a C-terminal arginine-glycine-rich (RGG) domain.

Based on these results, Foxo6os and human analogues thereof constitute a therapeutic target in cardiac disorders as well as in cardiac protection and/or cardiac regeneration.

The IncRNA Foxo6os was found to be down-regulated in skeletal muscle from insulin resistance mouse models and in palmitate stimulated mouse myoblast cells (Zhang et al., 2018). A medical use for Foxo6os, however, has not been postulated.

Accordingly, increasing the amount and/or expression of the IncRNA Foxo6os or an analogous IcnRNA has a therapeutic potential in medicine, particularly in the prevention or treatment of cardiac disorders and/or for cardiac regeneration.

A first aspect of the present invention relates to a nucleic acid molecule comprising
(i) the nucleotide sequence of SEQ ID NO: 1, 2, 3, 4 or 5 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii),
for use in medicine.

A specific embodiment of the invention is the nucleic acid molecule of SEQ ID NO: 1, which is human transcript AC093151.3 transcript 201 (human, ENST00000425554). This transcript is composed of three exons. Its genomic location is in the human genome (GRCh38/hg38) on chromosome 1:41,242,373-41,264,558 (22185 bp).

Thus, in certain embodiments, the invention relates to a nucleic acid molecule for use in medicine comprising
(i) the nucleotide sequence of SEQ ID NO: 1 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii).

A further specific embodiment is the nucleic acid molecule of SEQ ID NO: 2, which is human transcript AC093151.3 transcript 202 (human, NST00000445073).

Thus, in certain embodiments, the invention relates to a nucleic acid molecule for use in medicine comprising
(i) the nucleotide sequence of SEQ ID NO: 2 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii).

Still a further specific embodiment is the nucleic acid molecule of SEQ ID NO: 3, which is human transcript AC093151.3 transcript 203 (human, ENST00000422305).

Thus, in certain embodiments, the invention relates to a nucleic acid molecule for use in medicine comprising
(i) the nucleotide sequence of SEQ ID NO: 3 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii).

Still a further specific embodiment is the nucleic acid molecule of SEQ ID NO: 4, which is human transcript AC0933151.3 transcript 204 (human, ENST00000670398).

Thus, in certain embodiments, the invention relates to a nucleic acid molecule for use in medicine comprising
(i) the nucleotide sequence of SEQ ID NO: 4 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii).

Still a further specific embodiment is the nucleic acid molecule of SEQ ID NO: 5, which is the Foxo6os full-length mouse sequence (mouse, ENSMUST00000152384). This transcript is composed of two exons. Its genomic location is in the mouse genome (GRCm38/mm10) on chromosome 4: 120,291,350-120,303,892 (12542 bp).

Thus, in certain embodiments, the invention relates to a nucleic acid molecule for use in medicine comprising
(i) the nucleotide sequence of SEQ ID NO: 5 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii).

The invention also relates to a nucleic acid molecule, which is a functional fragment of a nucleotide sequence of SEQ ID NO: 1, 2, 3, 4 or 5. A functional fragment is understood as a fragment of any of the above sequences, which has at least partially, e.g. at least about 10%, at least about 30%, at least about 50%, at least about 70% and at least about 90% retained the physiological activity of the complete sequence as described in the present Examples. For example, a functional fragment may have a deletion of about 100 nt or less, about 50 nt or less, about 20 nt or less or about 10 nt or less at the 5' terminus and/or a deletion of about 100 nt or less, about 50 nt or less, about 20 nt or less or about 10 nt or less at the 3' terminus of the nucleotide sequence of SEQ ID NO: 1, 2, 3, 4 or 5.

The invention further relates to a nucleic acid molecule, which has an identity of at least about 70 %, at least about 80 %, at least about 90 % or at least about 99 % to the nucleotide sequence of SEQ ID NO: 1, 2, 3, 4 or 5 or a functional fragment thereof. The identity of a nucleic acid molecule with a reference nucleic acid molecule is determined over the whole length according to a known algorithm such as BLAST.

The invention further relates to a nucleic acid molecule, which is complementary to the nucleotide sequence of SEQ ID NO: 1, 2, 3, 4 or 5 or a functional fragment thereof or a nucleotide sequence, which has an identity to one of the sequences as indicated above. Complementarity is understood as the capacity of forming a base pair in a double-stranded nucleic acid molecule via hydrogen bonds, particularly a Watson-Crick base pair, e.g. between C and G and between A and T (or U). Further, in certain embodiments, complementarity is understood as complementarity over the whole length or substantially the whole length of two sequences.

The nucleic acid molecule of the present invention may be a DNA molecule, an RNA molecule, a modified nucleic acid molecule or any combination thereof. The nucleic acid molecule may be a single-stranded, double-stranded or partially single- and double-stranded.

In certain embodiments, the nucleic acid molecule is an RNA molecule, particularly an RNA molecule (i) or (ii) as indicated above, optionally including a 3'-modification such as a polyA tail and/or a 5'-modification such as a capping group. The RNA molecule may be single- or double-stranded.

The nucleic acid molecule may be a non-modified RNA molecule consisting of A, C, G and U building blocks. In further embodiments, the RNA molecule comprises at least one modified building block. The modified building block may be selected from a base-modified building block, a sugar-modified building block and/or a phosphate-modified building block.

In certain embodiments, the RNA molecule comprises at least one base-modified building block, e.g. wherein a naturally occurring RNA nucleobase, i.e. A, C, G or U is replaced by a different nucleobase. For example, a nucleobase U may be replaced by T.

In certain embodiments, the RNA molecule comprises at least one sugar-modified building block, wherein a naturally occurring RNA sugar, i.e. ribose is replaced by a different sugar or sugar analogue. For example, ribose may be replaced by an aza- or a carbacyclic analogue thereof, and/or a substituent on the ribose is modified. In certain embodiments, the 2'-OH substituent on the ribose is replaced by a substituent, which is different from OH. For example, the 2'-OH group is replaced by (i) a 2'-OR¹ group, wherein R¹ is C₁-C₄ alkyl, C₂-C₄ alkenyl or C₂-C₄ alkynyl optionally substituted by halo e.g. F, Cl or Br, OH or OC₁-C₄ alkyl, (ii) a 2'-NR²R³ group, wherein R² and R³ are independently selected from H, C₁-C₄ alkyl, C₂-C₄ alkenyl or C₂-C₄ alkynyl optionally substituted by halo e.g. F, Cl or Br, OH or OC₁-C₄ alkyl, or wherein R² and R³ are linked with each other, thereby forming a cyclic moiety, e.g. a 3-6 membered cyclic moiety, (iii) a 2'-halo group, e.g. F, Cl or Br or (iv) a 2'-SR⁴ group, wherein R⁴ is C₁-C₄ alkyl, C₂-C₄ alkenyl or C₂-C₄ alkynyl optionally substituted by halo e.g. F, Cl or Br, OH or OC₁-C₄ alkyl.

In certain embodiments, the RNA molecule comprises at least one backbone-modified building block wherein at least one internucleosidic phosphodiester group is replaced by a modified group e.g. a phosphoramidate or phosphorothioate group.

In certain embodiments, the RNA molecule is an RNA vector, e.g. a recombinant retrovirus vector comprising an RNA molecule (iii), i.e. complementary to an RNA molecule (i) or (ii) as indicated above. The RNA vector is suitable for gene expression in a prokaryotic cell, or in a eukaryotic cell, particularly in a mammalian cell and more particularly in a human cell. Thereby, an RNA molecule (i) or (ii) may be produced in a target cell. Suitable RNA vectors capable of expressing heterologous nucleic acids in a cell, e.g. in a mammalian cell, particularly in a human cell, are well known in the art.

In certain embodiments, the nucleic acid molecule is a DNA molecule, particularly a DNA molecule encoding an RNA molecule (i) or (ii) as indicated above. The DNA molecule may be single- or double-stranded. The DNA molecule may be in operative linkage with an expression control sequence, which may include a promotor and optionally further at least one further element such as an enhancer, a transcription factor binding side, etc. Typically, the expression control sequence is an expression control sequence which promotes expression of the nucleic acid molecule in a predetermined target cell, e.g. a prokaryotic cell or a eukaryotic cell, particularly in a mammalian cell and more particularly in a human cell or in vitro, whereby an RNA molecule encoded by the DNA molecule is obtained.

The DNA molecule may be a "naked" DNA molecule. Alternatively, it may be present on a vector, e.g. an expression vector suitable for transient or stable gene expression in a prokaryotic cell, or in a eukaryotic cell, particularly in a mammalian cell and more particularly in a human cell. The vector may be a non-viral vector, for example a plasmid or a viral vector, for example a recombinant adenovirus-associated vector (rAAV), a recombinant adenovirus vector. Suitable DNA vectors capable of expressing heterologous nucleic acids in a target cell, e.g. in a mammalian cell, particularly in a human cell, are well known in the art.

In certain embodiments, the nucleic acid molecule is conjugated to a heterologous moiety, e.g. a lipid, sugar or peptide moiety. The heterologous moiety may be conjugated to a base, to a sugar and/or to a phosphate group of the nucleic acid molecule. The heterologous moiety may be a moiety which improves targeting and/or cellular uptake, e.g. a lipid moiety such as cholesterol or a fatty acid, a saccharide or amino saccharide moiety such as a N-galactosamine containing moiety, a peptide or polypeptide moiety or a nucleosidic or nucleotidic moiety such as an aptamer. A heterologous moiety may be conjugated with the 5'- and/or 3'-terminus of the nucleic acid molecule by means of a covalent bond or a spacer.

A further aspect of the invention relates to a genome editing composition which is adapted for activating endogenous expression of a nucleic acid molecule of SEQ ID NO: 1, 2, 3, 4 or 5 or a nucleotide sequence which has an identity of at least about 70%, at least about 80%, at least about 90% or at least about 99% to the nucleotide sequence of SEQ ID NO: 1, 2, 3, 4 or 5 in a eukaryotic cell or organism, particularly a mammalian cell or organism, more particularly in a human cell or organism.

The genome editing composition is suitable for modifying the genome of a target cell in order to activate endogenous expression of an RNA molecule as indicated above. The modification may include modifying the endogenous expression control sequence of the target IncRNA and/or modifying the endogenous sequence encoding the target IncRNA. Modification of the target gene genome may comprise introducing a single- or double-stranded cut into the genome and inserting, deleting and/or substituting nucleic acid sequences of the target cell genome. In certain embodiments, the genome editing composition may comprise a genome-editing enzyme and optionally at least one corresponding genome-editing nucleic acid. For example, the genome editing enzyme may be a CRISPR/Cas enzyme, e.g. a CRISPR/Cas 9 or 13 enzyme or any variant thereof, a transcription activator-like effector-based nuclease (TALEN), a zinc finger nuclease protein, a recombinase, a meganuclease or an Argonaute protein, e.g. of the bacterium Thermus thermophiles (TtAgo). The genome-editing nucleic acid may be a sgRNA for use with a CRISPR/Cas enzyme. Further, the genome editing composition may comprise a nucleic acid molecule coding for a genome-editing enzyme as indicated above.

Suitable transfection techniques for introducing proteins or nucleic acids into the eukaryotic target cells are well known in the art and include lipofection, electroporation, e.g. nucleofection, Ca-phosphate or virus-based methods.

A further aspect of the invention relates to a pharmaceutical preparation comprising a nucleic acid molecule as described above, a genome editing composition as described above, together with a pharmaceutically acceptable carrier.

The nucleic acid molecule, the genome editing composition and the pharmaceutical preparation of the present invention (in the following specified as "active agent") are suitable for use in medicine including human and veterinary medicine, particularly for the prevention and/or treatment of a cardiac disorder and/or for cardioprotection and/or for cardioregeneration.

The term "prevention" in the context of the present invention relates to the administration of the active agent to a patient, who is known or suspected to have an increased risk of developing a certain disorder. The term "treatment" in the context of the present invention relates to the administration of the active agent to a patient, who has already developed signs and/or symptoms of a certain disorder. The term "cardioprotection" relates to the administration of the active agent to a patient, who is at risk or suspected being at risk of suffering cardiac damage, with the purpose of at least partially preventing the occurrence of cardiac damage. The term "cardioregeneration" relates to the administration of the active agent to a patient, who has suffered cardiac damage, with the purpose of removing and/or ameliorating the cardiac damage. The term "patient" relates to a subject in need of administration of the active agent of the invention in the field of human or veterinary medicine. In specific embodiments, the patient is a human patient.

In certain embodiments, the active agent of the present invention is useful in the prevention or treatment of a cardiac disorder, particularly of cardiac hypertrophy-associated disorder. For example, the active agent is useful in the prevention or treatment of contractile dysfunction, cardiac decompensation, heart failure, e.g. diastolic heart failure, or atrial fibrillation, or for the prevention or treatment of cardiac remodeling after myocardial infarction, myocarditis, valvular heart diseases such as aortic stenosis or mitral valve insufficiency, genetic cardiac disorders with cardiac hypertrophy, e.g. hypertrophic non-obstructive and obstructive cardiomyopathy or Fabry disease.

In certain embodiments, the active agent is useful for cardiac protection and/or cardiac regeneration.

The active agent is useful for administration to patients selected from
(i) patients having an increased risk for developing heart failure,
(ii) patients suffering from (congestive) heart failure, e.g. patients having an increased risk of heart failure progression,
(iii) post-myocardial infarction patients,
(iv) patients with congenital heart diseases associated with cardiac hypertrophy, such as pulmonal vein stenosis, atrial or ventricular septum defects and/or
(v) patients suffering from hypertrophic cardiomyopathy.

A further aspect of the invention relates to a cell, organ or a non-human organism transfected or transformed with a nucleic acid molecule or a genome editing composition as described above. In certain embodiments, the cell is a cardiomyocyte, particularly a human cardiomyocyte or a precursor cell thereof.

Still a further aspect of the present invention is a cell, organ or a non-human organism having an increased endogenous expression of a nucleic acid molecule of SEQ ID NO: 1, 2, 3, 4 or 5 or of a nucleotide sequence which has an identity of at least about 70%, at least about 80%, at least about 90% or at least about 99% to the nucleotide sequence of SEQ ID NO: 1, 2, 3, 4 or 5 compared to a wild-type cell, organ or organism. For example, the endogenous expression may be increased by at least about 10%, at least about 50% or at least about 100% as measured by quantitative real-time polymerase chain reaction (qPCR) or quantitative RNA fluorescence in situ hybridization (RNA-FISH).

The active agent of the invention may be administered as a pharmaceutical preparation comprising a pharmacologically acceptable carrier. Administration may be carried out by known methods, wherein the active agent is introduced into the desired target cell or organ of the subject to be treated.

For pharmaceutical applications, the active agent may be in the form of a solution, e.g. an injectable solution, emulsion, inhalation, suspension or the like.

The active agent may be administered in any suitable way, e.g. parenterally, in particular by injection such as subcutaneous, intramuscular, intravenous or intra-arterial injection, or, infusion, by oral or inhalative intake and/or by dermal or transdermal application. The carrier may be any suitable pharmaceutical carrier. Preferably, a carrier is used which is capable of increasing the efficacy of nucleic acid molecules to enter the target cells. Suitable examples of such carriers are nanoparticles, liposomes, e.g. cationic liposomes, or predesigned exosomes.

The active agent is administered in a pharmaceutically effective dosage depending on the route of administration and the type or severity of the disease.

The active agent may be administered as a monotherapy or in combination with a further different medicament, particularly a medicament suitable for the prevention or treatment of cardiac disorders or fibrotic disorders as described above.

Examples of further medicaments suitable for the prevention or treatment of cardiac disorders are angiotensin-modulating agents, β-blockers, diuretics, aldosterone antagonists, vasodilators, ionotrophic agents, statins, or neprilysin-inhibitors or combinations thereof, e.g. a combination of a neprilysin-inhibitor, e.g. sacubitril, with an angiotensin-II-receptor blocker, e.g. valsartan. In certain embodiments, the further medicament is entresto.

In further aspects, the invention encompasses determining the amount and/or activity of certain physiological parameters in the subject to whom the active agent is administered before, during and/or after administration. This concomitant diagnostic procedure and the reagent used for this diagnostic procedure may provide assistance for the medical use as described above. For example, the diagnostic procedure may provide assistance in risk assessment, patient stratification, monitoring of treatment course and/or posttreatment control.

Still a further aspect of the present invention is a method of detecting, diagnosing or monitoring a cardiac disorder comprising detecting a nucleic acid molecule as described above, particularly an endogenous IncRNA molecule and/or an exogenously introduced RNA molecule (i) or (ii) as described above.

Still a further aspect of the present invention is a reagent for detecting, diagnosing or monitoring a cardiac disorder comprising a reagent or reagent combination for detecting, diagnosing or monitoring a cardiac disorder comprising detecting a nucleic acid molecule as described above, particularly an endogenous IncRNA molecule and/or an exogenously introduced RNA molecule (i) or (ii) as described above.

In certain embodiments, the invention encompasses determining the amount and/or activity of an IncRNA as described above. In further embodiments, the invention encompasses determining the amount and/or activity of cardiac markers such as NT-ProBNP and/or troponin T during the course of therapy. In further embodiments, the invention encompasses determining the amount and/or activity of physiological targets of the IncRNA such as CIRBP and RBM3.

The determination of the above parameters may be carried out in body fluid samples such as blood, plasma or serum or in tissue samples according to known methods at the nucleic acid and/or protein level and may provide useful diagnostic information, e.g. on the course and/or success of the treatment.

Further, the invention shall be explained in more detail by the following examples and figures:

### Examples

### Example 1

### Identification of Foxo6os IncRNA as a therapeutic target.

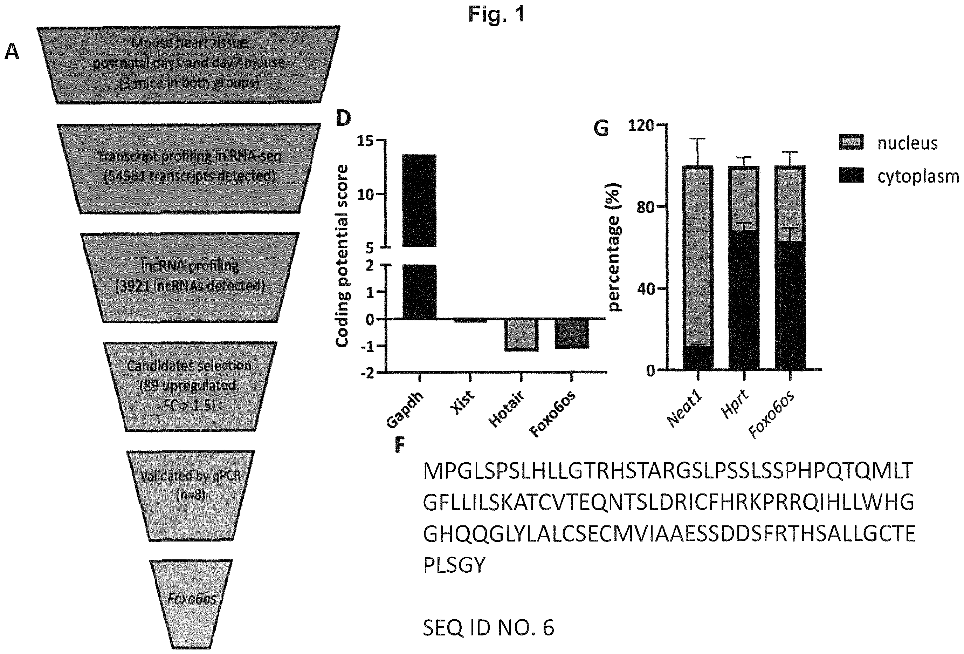

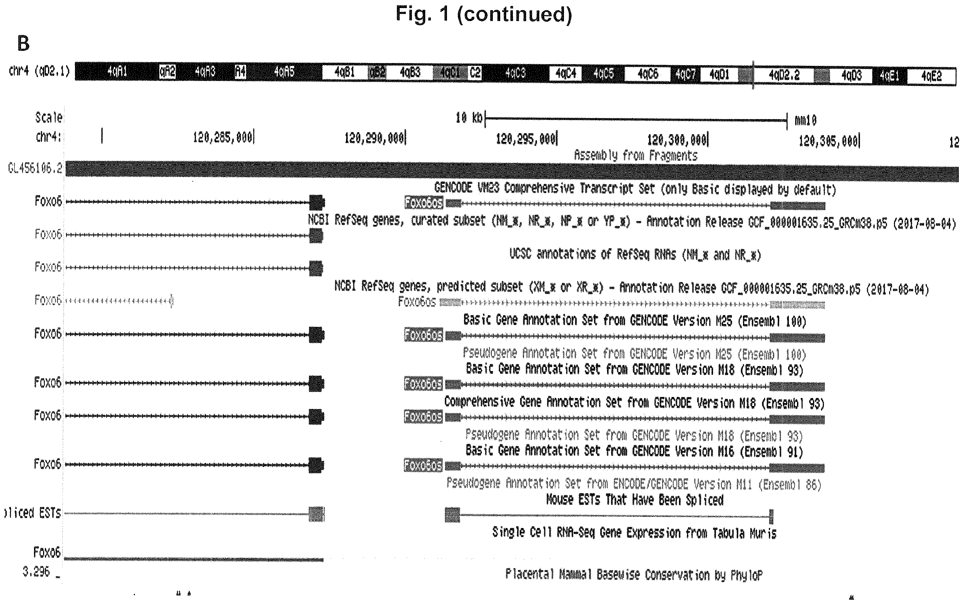

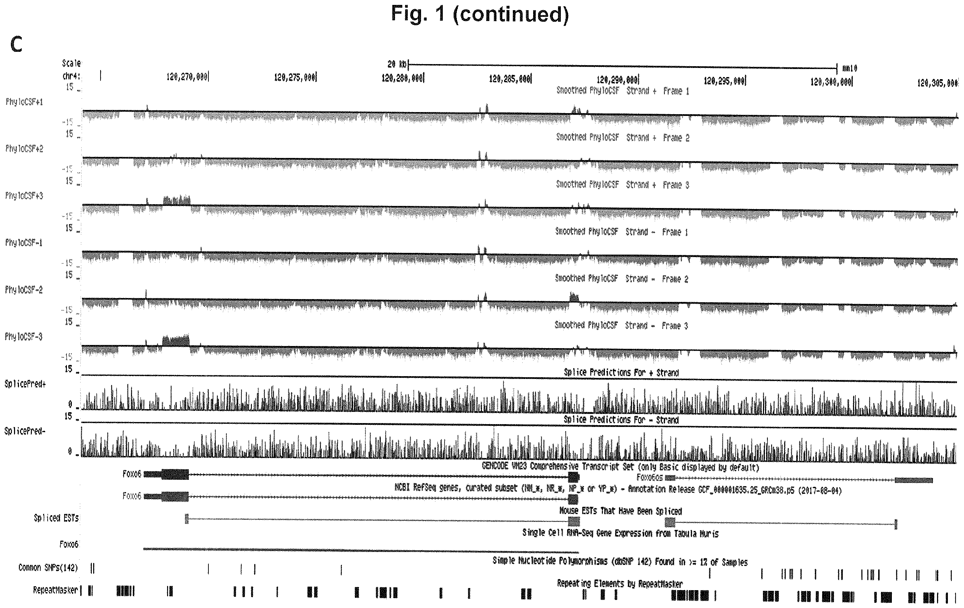

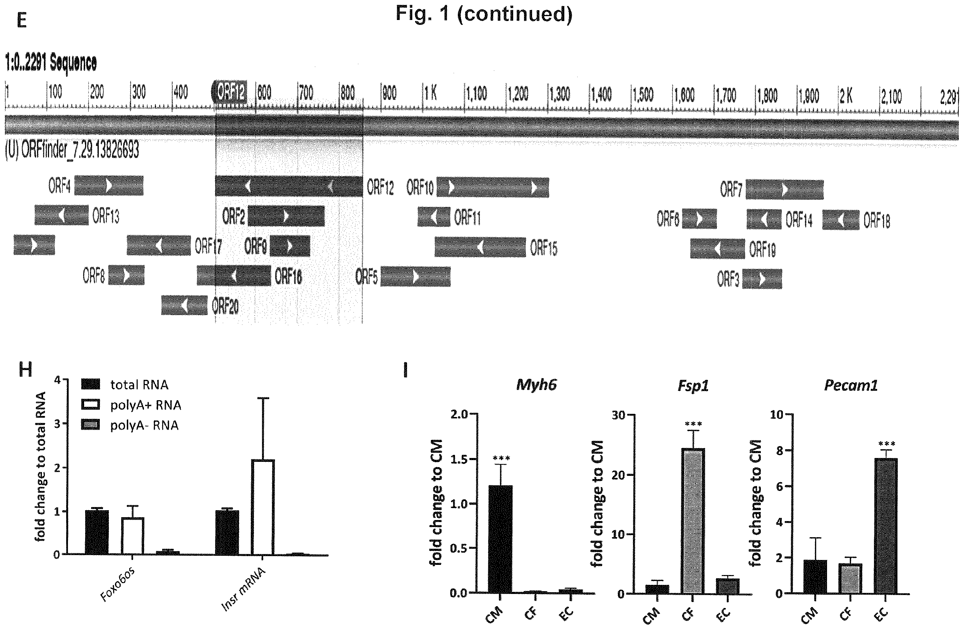

Using next generation sequencing technology for a genome-wide IncRNA screen in the developing mouse heart identified Foxo6os, a poly(A)⁺ IncRNA, as a potential therapeutic target. The results are shown in Figure 1 :
(A) Pipeline to select candidates from RNA-seq data. (B) Genomic locus of Foxo6os in mouse (GRCm38/mm10) represented in UCSC Genome Browser. (C) Sequence conservation and coding potential of Foxo6os was analyzed by (C) PhyloCSF and (D) coding potential calculator 2(CPC2). Note that the positive peak only appears in Foxo6 but not in Foxo6os. For CPC2, Hotair, Xist and Gapdh were calculated as positive controls. (E) The cDNA sequence of Foxo6os was analyzed by NCBI ORF finder, and the predicted amino acid translated from the largest open reading frame (ORF) in Foxo6os was shown in (F) (blue box, ORF 12). (G) Subcellular distribution of Foxo6os revealed cytoplasmic localization in cardiomyocyte-like HL-1 cells. Neat1 and Hprt expression levels were measured as positive controls. (H) Cell-specific markers for each cell type from heart fractionation were validated by qPCR to determine the cell purity. Myh6 is the marker for cardiomyocyte (CM), Fsp1 is the marker for cardiac fibroblast (CF) and Pecam1 is the marker for endothelial cells (EC). (I) PolyA tail was detected in Foxo6os with Oligotex polyA tail kit and validated by qPCR. Data are mean ± SEM (N ≥ 3 independent experiments). ***P < 0.005 as calculated from student's t-test.

### Example 2

### Expression of Foxo6os IncRNA in mice under physiological and disease conditions.

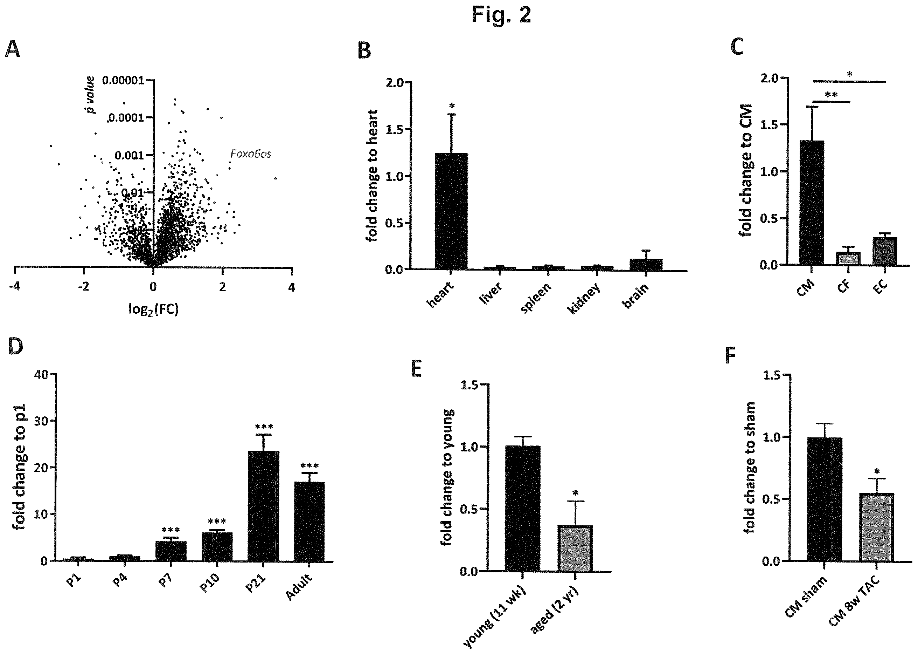

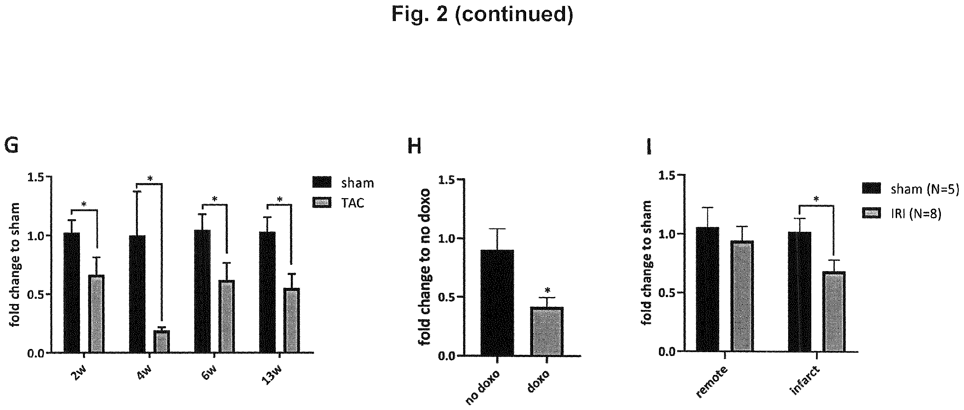

Foxo6os expression was analyzed in mouse hearts. It was found that the expression increased under physiological conditions but decreased under disease conditions. The results are shown in Figure 2:
(A) Volcano plot of differentially regulated IncRNAs from RNA-seq data of postnatal day 1 vs day 7 mouse hearts. Foxo6os is indicated in red. FC: fold change. (B) Validation of Foxo6os expression by qPCR in mouse organ panels (N=5). (C) Expression profile of Foxo6os was confirmed in different cell types isolated from mouse heart via qPCR, including cardiomyocyte (CM), cardiac fibroblast (CF) and endothelial cells (EC). (D) Validation of Foxo6os expression by qPCR from the mouse heart (N=5) of different ages as indicated. P: postnatal day. (E) Expression level of Foxo6os in aging mouse hearts (2 years old) and young mouse hearts (11 weeks old) (N=4 or more). The expression of Foxo6os was further measured in (F) cardiomyocyte isolated from 8 weeks TAC heart, and (G) transverse aortic constriction (TAC) mouse whole hearts (N=6) for different weeks as indicated. In addition to TAC disease model, the expression of Foxo6os was also measured in (H) doxorubicin (doxo, 5 mg/kg)-treated hearts (N=9) for 5 weeks and (I) ischemia-reperfusion injury (IRI, ischemia for 60 min and reperfusion for 24 h) heart (N=5 or more). Data are mean ± SEM (N ≥ 3 independent experiments). *P < 0.05; **P < 0.01; ***P < 0.005 as calculated from student's t-test.

### Example 3

### Inhibition of Foxo6os expression in cardiomyocytes

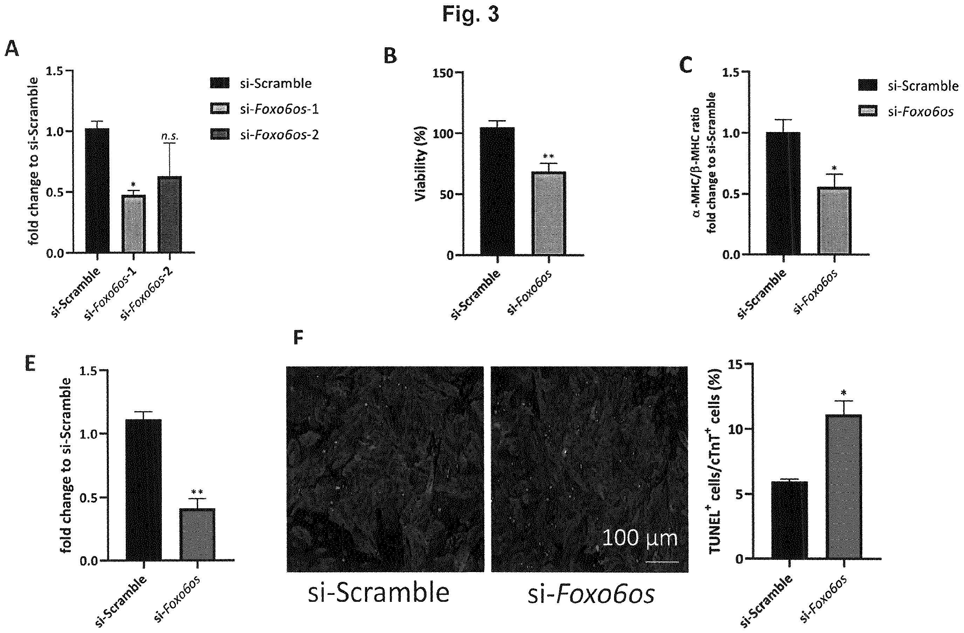

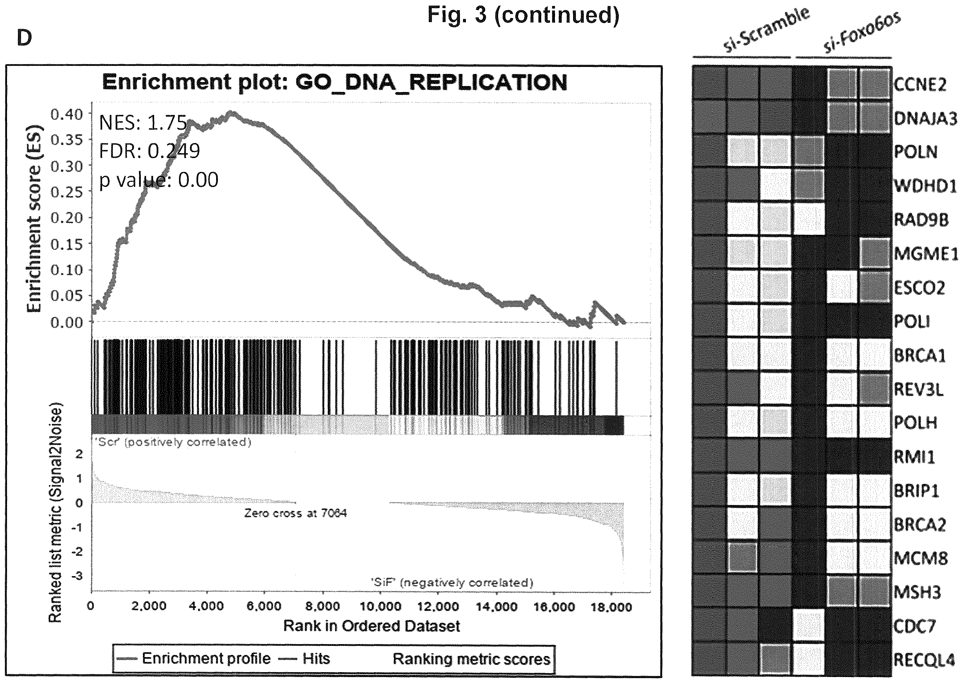

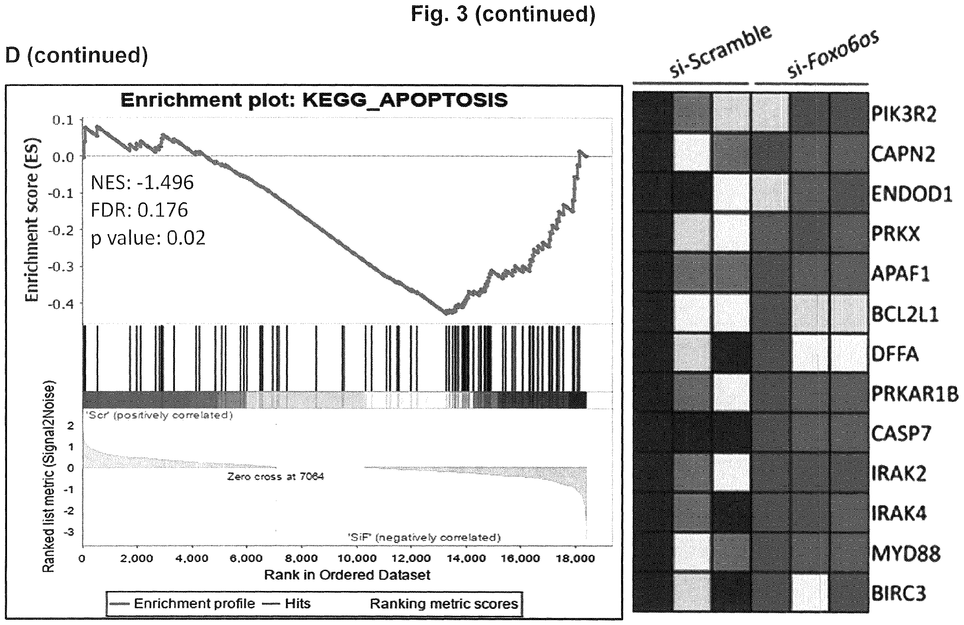

It was found that inhibition of Foxo6os expression in cardiomyocytes by an siRNA leads to an apoptosis phenotype. The results are shown in Figure 3:
(A) Two siRNAs against *Foxo6os* were designed and the knockdown efficiency was tested in HL-1 by qPCR. The si-*Foxo6os*-1 was later used for all the inhibition experiments. (B) HL-1 viability was measured by WST-1 assay after treated with si-Scramble or si*-Foxo6os.* (C) The expression of α-/*β-MHC* were measured by qPCR after HL-1 treated with si-Scramble or si-*Foxo6os,* and the ratio of *α-*/*β-MHC* was plotted. (D) GSEA analysis and heatmap from the HL-1 RNA-seq data of "si-Scramble" compared to "si-*Foxo6os*"*.* (E) Treated si-*Foxo6os*-*1*/si-Scramble to neonatal mouse cardiomyocytes (NMCMs) and the expression of *Foxo6os* was measured by qPCR (F) Apoptosis rate of NMCMs after si-*Foxo6os*/si-Scramble treatment was measured by TUNEL assay. Green: TUNEL-488; red: cardiac troponin T; blue: DAPI. Data are mean ± SEM (N ≥ 3 independent experiments). *P < 0.05; **P < 0.01; n.s.: not significant as calculated from student's t-test.

### Example 4

### Knockout of Foxo6os expression in HL-1 cells

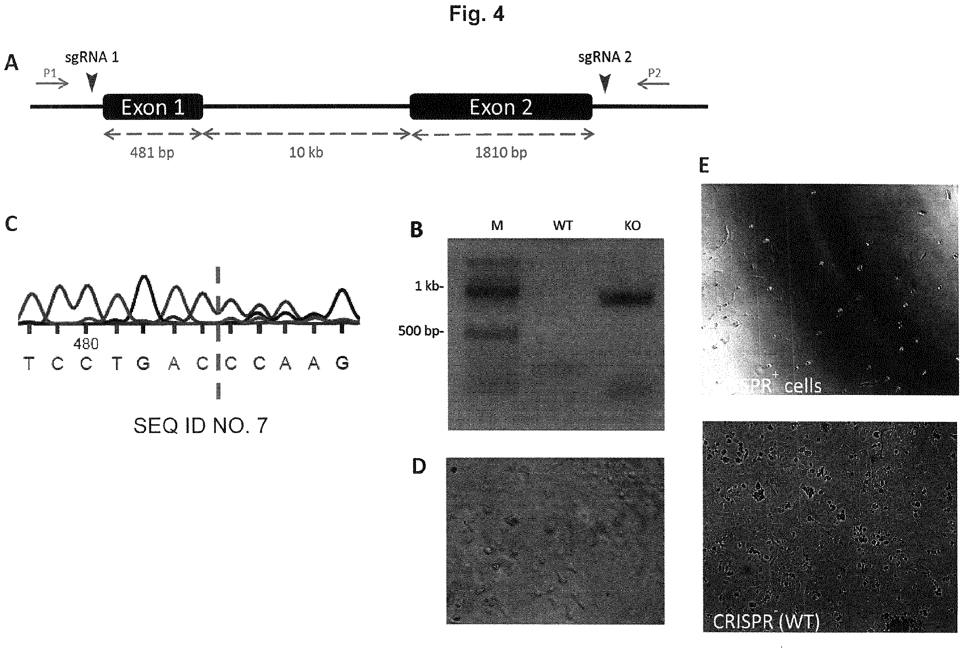

Foxo6os expression in HL-1 cells were knocked out by CRISPR/Cas9. It was found that knockout of a Foxo6os expression impairs cell proliferation. The results are shown in Figure 4:
(A) Scheme of *Foxo6os* exons, CRISPR/Cas9 sgRNAs and PCR primers for genotyping. The two sgRNAs work synergistically to delete the whole *Foxo6os* gene locus. P1 and P2 are primers for genotyping. (B) Deletion of *Foxo6os* locus was validated by electrophoresis of PCR products. M: marker, WT: wildtype, KO: knockout. (C) Sequencing chromatograms showed the mutant junction site (indicated by red dash line) of CRISPRed HL-1 cells gDNA. (D) The β-gal staining (blue) was colocalized with dTomato positive cells, which meant the cells were transfected by CRISPR/Cas9 plasmid. (D) The HL-1 cells were transfected with dTomato-CRISPR/Cas9 plasmid for 48 h and sorted by dTomato fluorescence, the dTomato positive and negative cells were then cultured for 2 weeks.

### Example 5

### Overexpression of Foxo6os in HL-1 cells via CRISPR/dCas activation

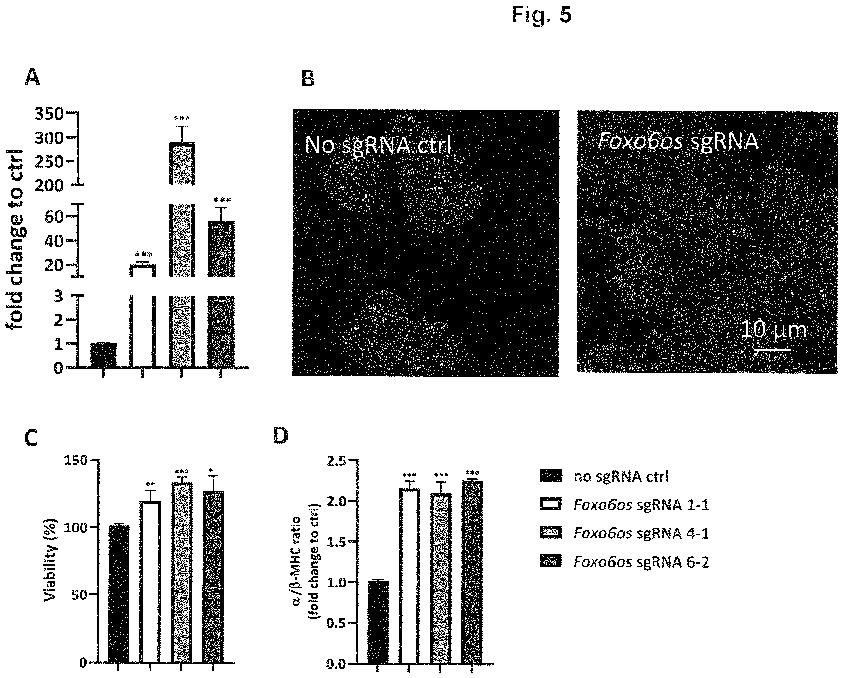

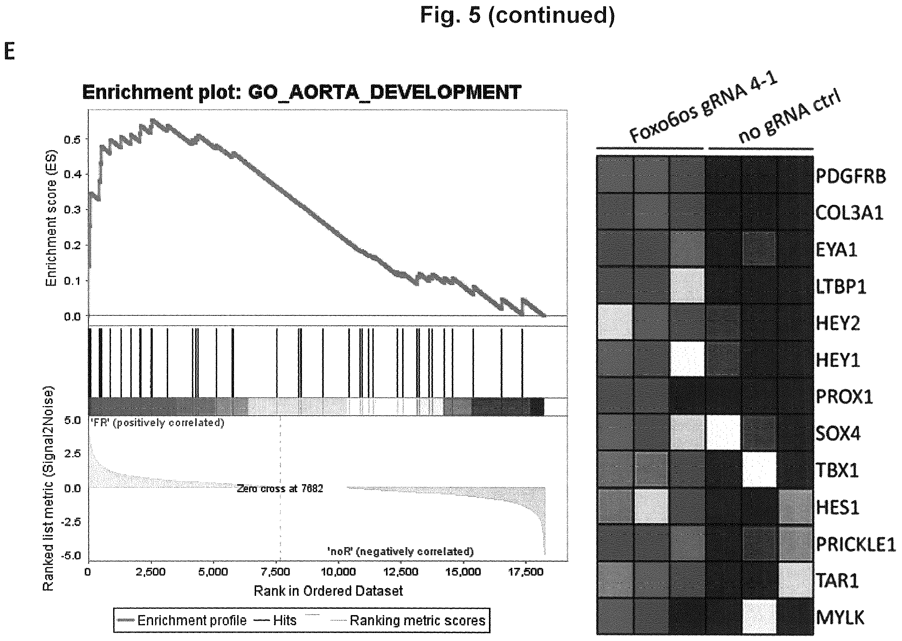

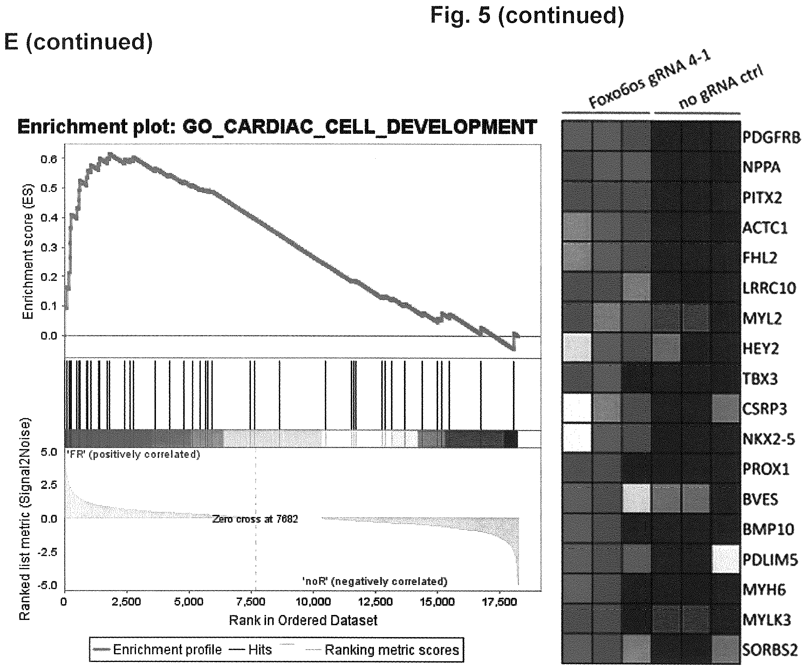

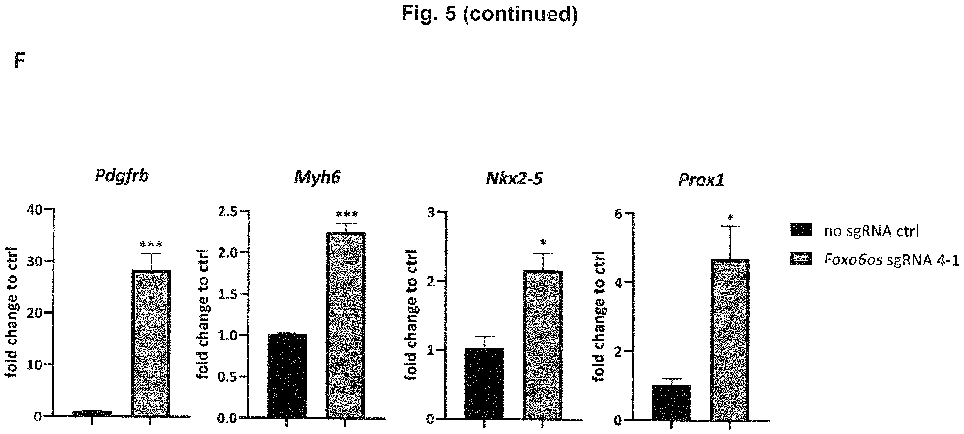

Using the CRISPRa system, Foxo6os was overexpressed in HL-1 cells via CRISPR/dCas activation. The results are shown in Figure 5:
Three different sgRNAs were used to activate *Foxo6os* expression and validated by (A) qPCR and (B) RNA FISH. Red: *Foxo6os* probe, Blue: nucleus (DAPI). Both of the (C) cell viability and (D) ratio of *α*/*β-MHC* are increased after overexpression of *Foxo6os.* (E) GSEA analysis and heatmap from the RNA-seq of "*Foxo6os* sgRNA 4-1 group" compared to "no sgRNA ctrl group". (F) Important genes that selected from GSEA analysis were further validated by qPCR. Data are mean ± SEM (N ≥ 3 independent experiments). *P < 0.05; **P < 0.01; ***P < 0.005 as calculated from student's t-test.

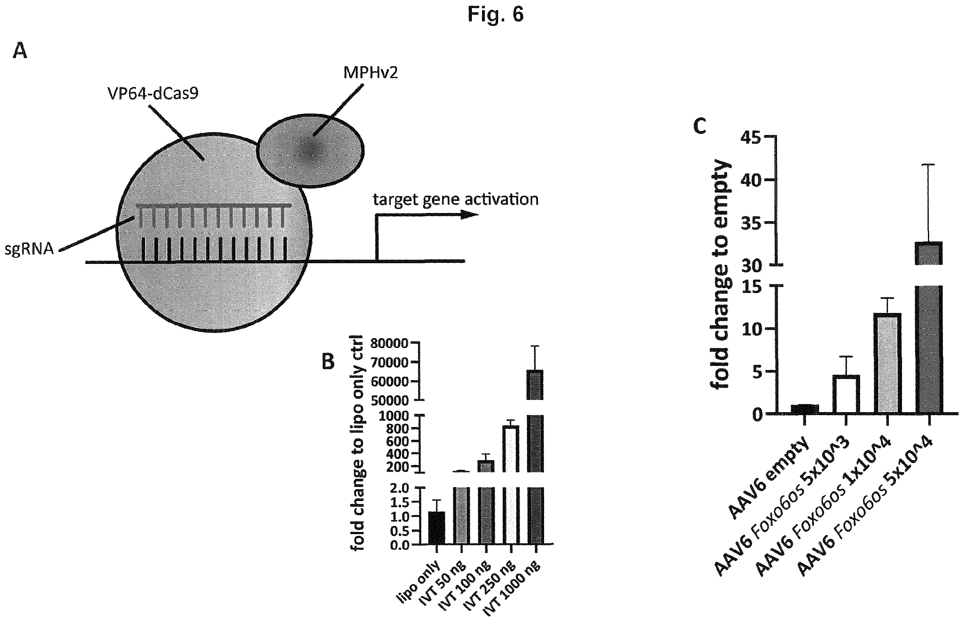

Overexpression of Foxo6os could also be carried out both endogenously and exogenously. The results are shown in Figure 6:
(A) Scheme of how CRISPRa system works. *Foxo6os* was also successfully overexpressed by (B) transfection of *in vitro* transcripted (IVT) *Foxo6os* RNA into HL-1 cells and (C) adeno-associated virus serotype 6 (AAV6)-mediated overexpression in NMCMs.

### Example 6

### Identification of human transcript AC093151.3 as a potential locus-conserved transcript.

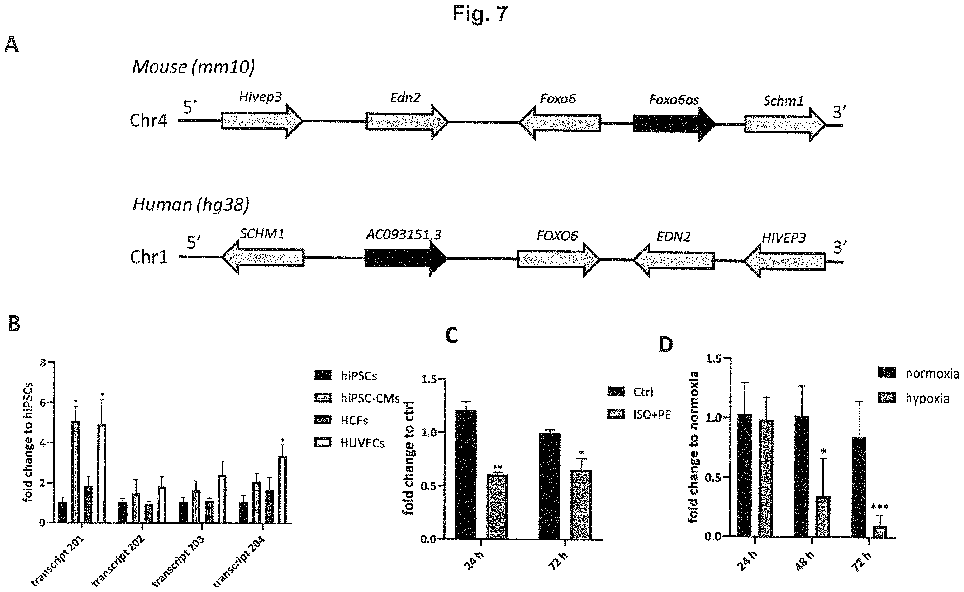

Human transcript AC093151.3 was identified as a locus-conserved transcript for mouse Foxo6os. The results are shown in Figure 7:
(A) Scheme of mouse chromosome 4 and human chromosome 1. The location of *Foxo6os,* its potential human homolog *AC093151.3* and their neighboring genes were plotted. The graph was not plotted to scale. (B) The expression of four AC093151.3 transcripts was validated via qPCR in different human cell lines, hiPSCs, hiPSC-CMs, HCFs and HUVECs. The expression of *AC093151.3* transcript 201 was further measured by qPCR in (C) hiPSC-CMs treated with and without ISO+PE treatment (100 µM) for 24 h or 72 h and (D) hiPSC-CMs under normoxia and 0.1% O₂ hypoxia condition for 24 h to 72 h. Data are mean ± SEM (N ≥ 3 independent experiments). **P < 0.05; **P < 0.01; ***P < 0.005* as calculated from student's t-test. hiPSC-CM: human induced pluripotent stem cell-derived cardiomyocyte, HCFs: human cardiac fibroblasts, HUVECs: human umbilical vein endothelial cells. ISO: isoproterenol, PE: phenylephrine.

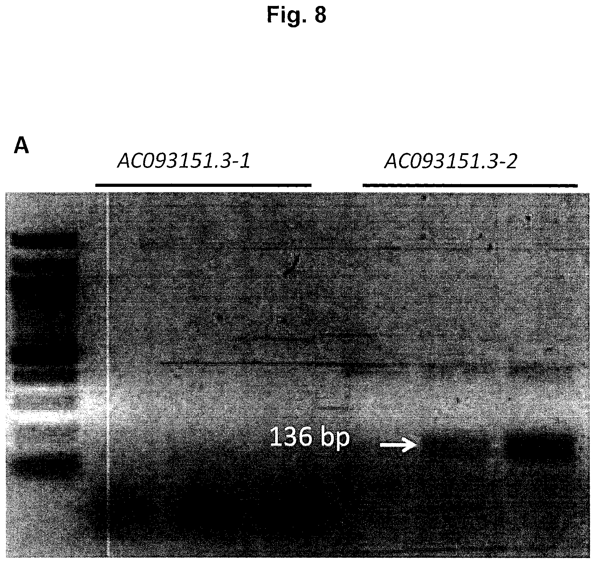

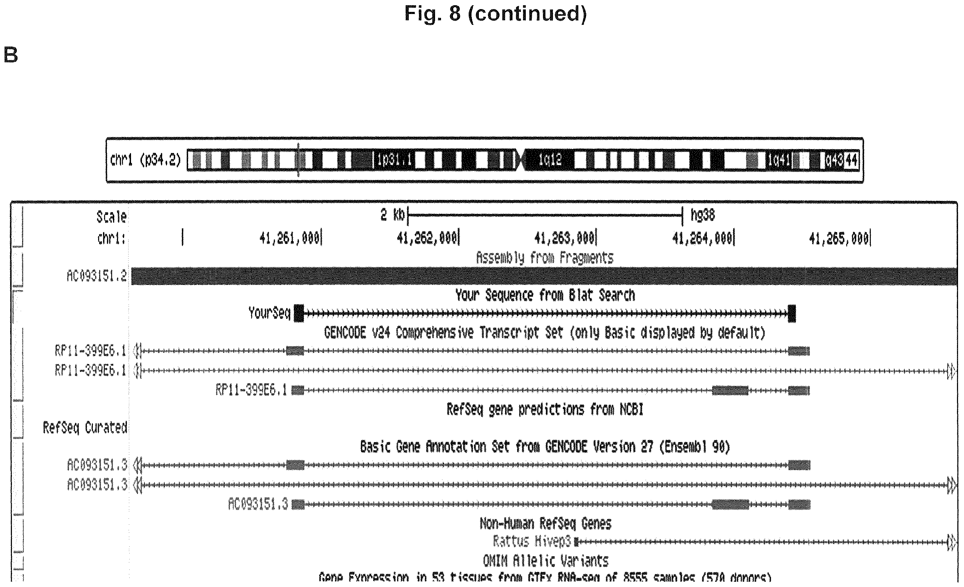

Further, the human AC093151.3 transcript was confirmed via gel electrophoresis and sequencing. The results are shown in Figure 8:
(A) Two different primers were designed to detect *AC093151.3* transcript 201 in hiPSC-CMs and the size of PCR product was indicated. (B) The *AC093151.3* PCR product was extracted, sequenced and BLASTed in UCSC genome browser (shown as "Your Seq").

### Example 7

### Transcription factors of Foxo6os

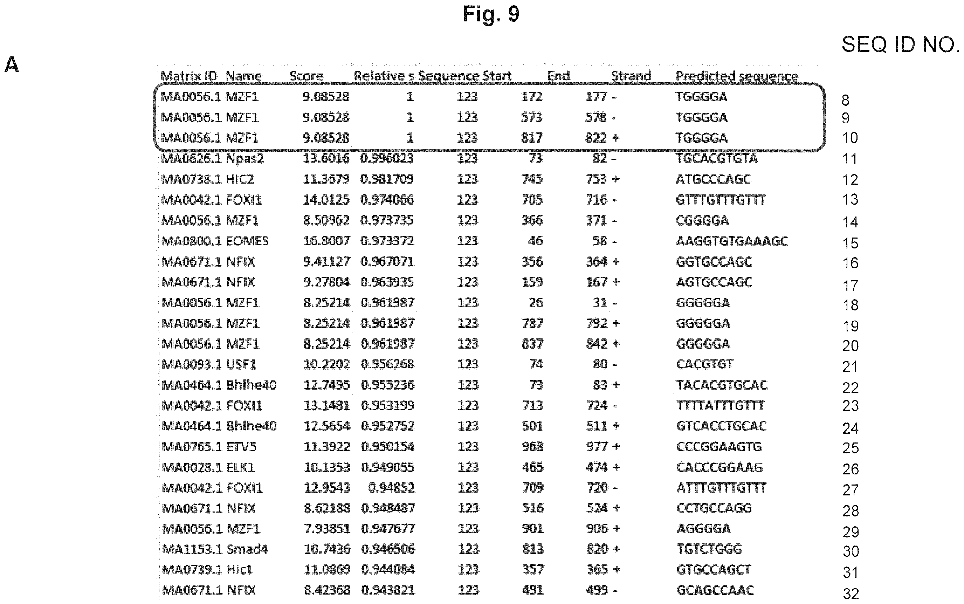

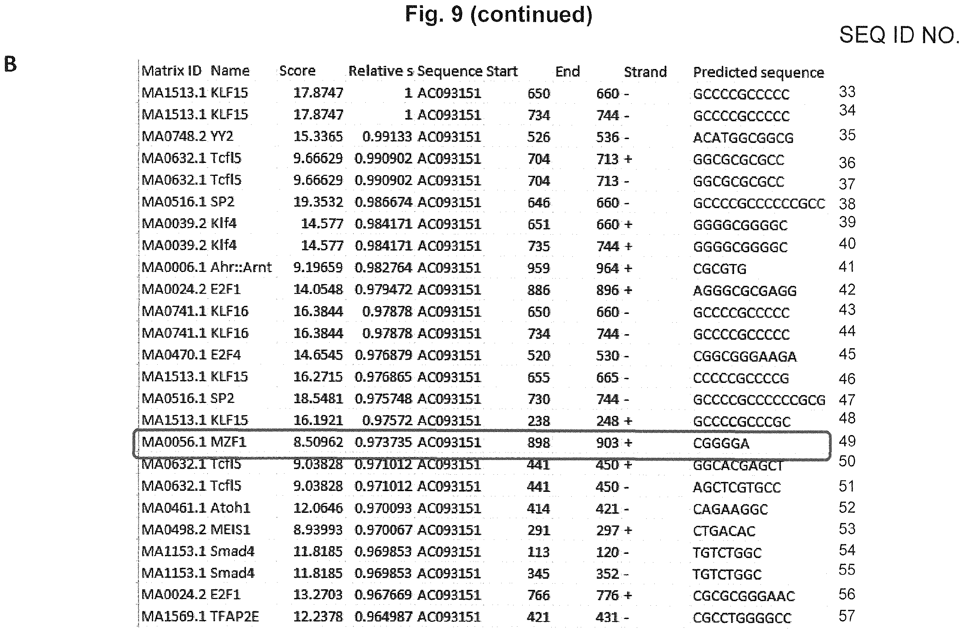

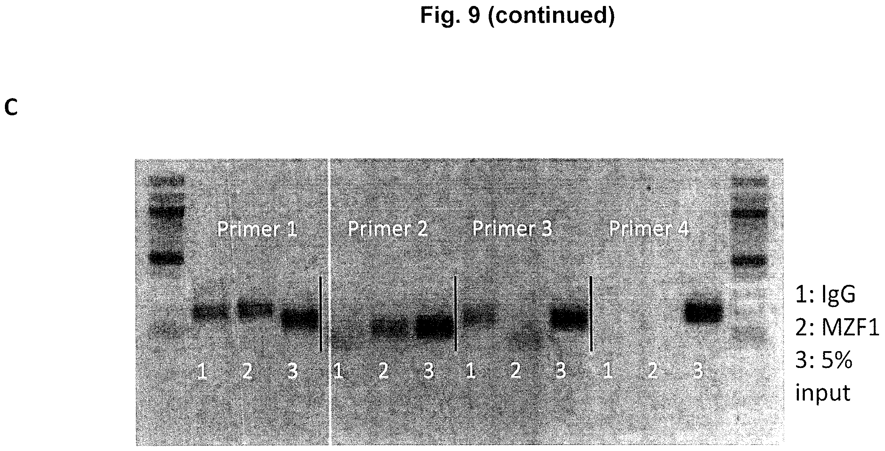

Lists of potential transcription factors that bind to promoter of mouse *Foxo6os* and human *AC093151.3* are shown in Figure 9:
The sequence of mouse *Foxo6os* promoter and human *AC093151.3* promoter (1 kp upstream of the transcription start site) were extracted from Ensembl and analyzed by JASPAR, among all of the 1011 TFs, 163 TFs were predicted for (A) mouse *Foxo6os* and 344 TFs were predicted for (B) *human AC093151.3.* Note that only the top 25 TFs were shown in the list and Mzf1 was indicated in red box. (C) Chromatin immunoprecipitation (ChIP) was performed against Mzf1 and control IgG followed by qPCR and then the qPCR product was validated by gel electrophoresis.

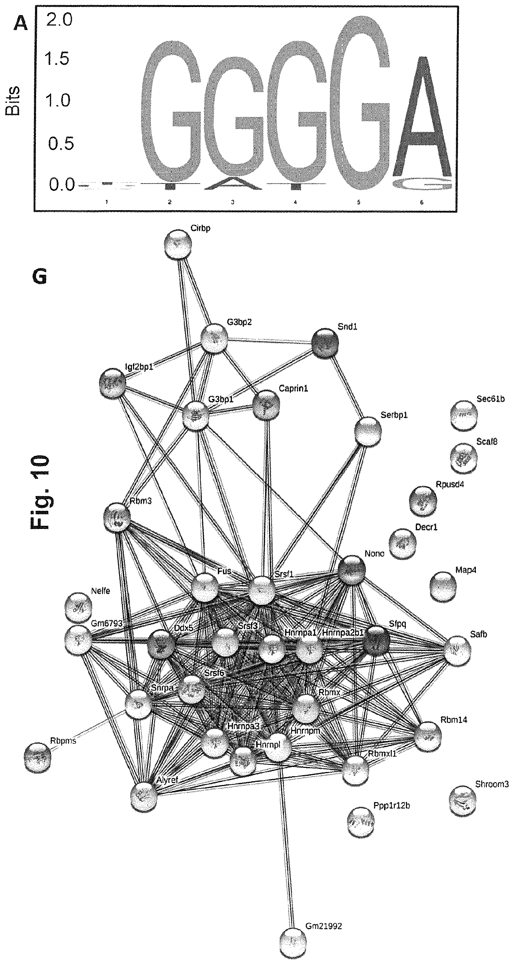

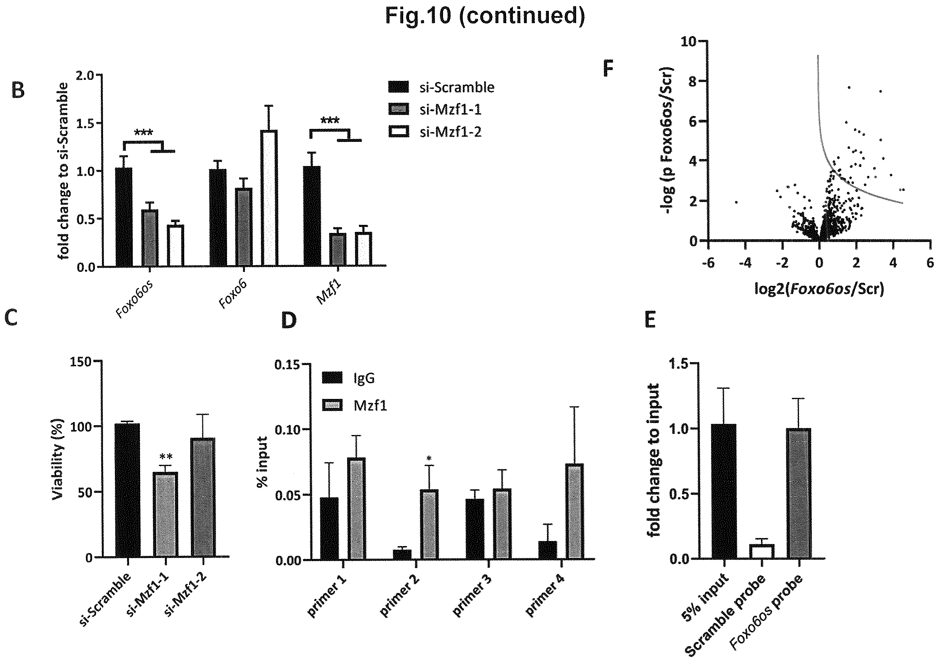

The transcription factor Mzf1 was shown to be essential for transcription of Foxo6os. The results are shown in Figure 10:
(A) Predicted transcription factor Mzf1 binding site by JASPAR. (B) The expression of *Foxo6os, Foxo6* and *Mzf1* were measured by qPCR after treatment of siRNA against Mzf1 in HL-1 cells. (C) HL-1 viability was measured by WST-1 assay after treated with si-Scramble or si*-Mzf1.* (D) Chromatin immunoprecipitation (ChIP) was performed against Mzf1 and control IgG followed by qPCR with primers specific to the *Foxo6os* promoter region in HL-1 cells. (E) RNA pulldown (N=6) and qPCR was performed in HL-1 to detect the protein candidates that potentially interact with *Foxo6os.* (F) The proteins/peptides that were detected by mass spectrometry was shown as dot plot. The red curve indicated the p value (<0,05) and q value (<0,05). Only the candidates that meet the selection criteria were further analyzed and the two green dots are Cirbp and Rbm3. (G) The 37 protein candidates were further analyzed by STRING and shown as protein-protein interaction network. Unless indicated individually, the data are mean ± SEM (N ≥ 3 independent experiments). **P* < *0.05; **P < 0.01; ***P < 0.005* as calculated from student's t-test.

### Example 8

### Foxo6os knockout mouse model

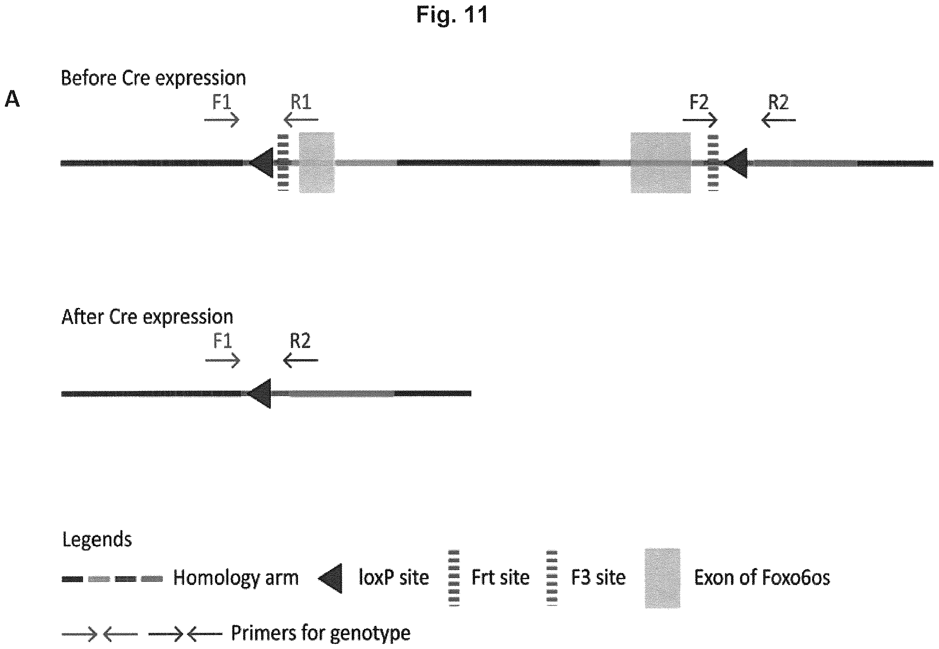

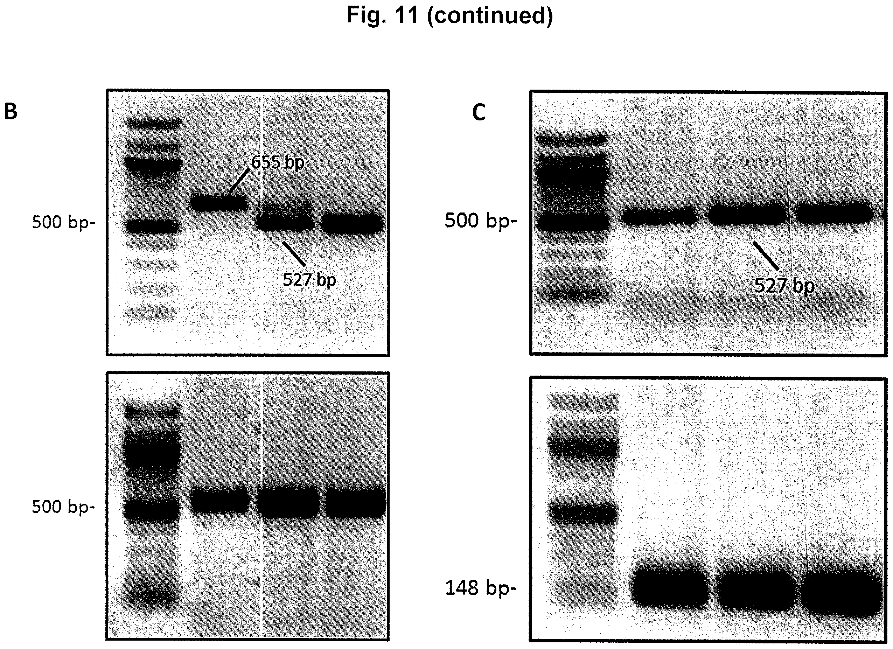

A Foxo6os knockout mouse model was generated via Cre-LoxP recombination. The experimental protocol and the results are shown in Figure 11:
(A) Scheme of *Foxo6os* gene locus and loxP site. Both exons of *Foxo6os* will be deleted after recombinase Cre induction. The graph was not plotted to scale. (B) Gel electrophoresis showed the different genotypes after PCR with primer set F1+R1 for *Foxo6os* floxed/Myh6-Cre mice. Upper panel (from left to right): homozygous floxed (product size: 655 bp), heterozygous floxed (product size: 527/655 bp) and wild type (product size: 527 bp). Lower panel: genotype of Myh6-Cre (product size: 595 bp). (C) Gel electrophoresis showed the genotype for *Foxo6os* floxed/CMV-Cre mice. After CMV-Cre induction, the whole *Foxo6os* gene locus was deleted. The representative gel images shown here are heterozygous mice with wildtype allele (527 bp, primer F1+R1) and knockout allele (148 bp, primer F1+R2).

### List of references

1. Barry, S.P.; Townsend, P.A. (2010). What causes a broken heart-Molecular insights into heart failure. Int Rev Cell Mol Biol 284, 113-179.
2. Datta, S.R.; Brunet, A.; Greenberg, M.E. (1999). Cellular survival: a play in three Akts. Genes Dev. 13, 2905-2927.
3. DeBosch, B.J.; Muslin, A.J. (2008). Insulin signaling pathways and cardiac growth. J Mol Cell Cardiol. 44, 855-864.
4. Doppler, S.A.; Werner, A.; Barz, M.; Lahm, H.; Deutsch, M.-A., Dreßen, M.; Schiemann, M.; Voss, B.; Gregoire, S.; Kuppusamy, R.; Wu, S.M.; Lange, R.; Kranke, M. (2014). Myerloid Zinc Finger 1 (Mzf1) Differentially Modulates Murine Cardiogenesis by Interacting with an Nkx2.5 Cardiac Enhancer. PLoS ONE 9 12: e113775.
5. Frescas, D.; Valenti, L.; Accili, D. (2005). Nuclear trapping of the forkhead transcription factor FoxO1 via Sirt1-dependent deacetylation promotes expression of glucogenetic genes. J Biol Chem. 280, 20589-20595.
6. Glas, D.J. (2010). PI3 kinase regulation of skeletal muscle hypertrophy and atrophy. Curr Top Microbiol Immunol. 346, 267-278.
7. Hill, J.A.; Olson, E.N (2008). Cardiac Plasticity. N Engl J Med. 358, 1370-1380.
8. McMullen, J.R.; Shioi, T.; Huang, W.Y.; Zhang, L.; Tarnavski, O.; Bisping, E.; Schinke, M.; Kong, S.; Sherwood, M.C.; Brown, J. et al. (2004). The insulin-like growth factor 1 receptor induces physiological heart growth via the phosphoinositide 3-kinase (p110alpha) pathway. J Biol Chem. 279, 4782-4793.
9. Ni, Y.G.; Berenji, K.; Wang, N.; Oh, M.; Sachan, N.; Dey, A.; Cheng, J.; Lu, G.; Morris, D.J.; Castrillon, D.H. et al. (2006). Foxo transcription factors blunt cardiac hypertrophy by inhibiting calcineurin signaling. Circulation. 114, 1159-1168.
10. Ronnebaum, S.M.; Patterson, C. (2010). The foxO family in cardiac function and dysfunction. Annu Rev Physiol. 72, 81-94.
11. Skurk, C.; Izumiya, Y.; Maatz, H.; Razeghi, P.; Shiojima, I.; Sandri, M.; Sato, K.; Zeng, L.; Schiekofer, S.; Pimentel, D. et al. (2005). The FOXO3a transcription factor regulates cardiac myocyte size downstream of AKT signaling. J Biol Chem. 280, 20814-23.
12. Zhang, N.; Zhou, Y.; Yuan, Q.; Gao, Y.; Wang, Y.; Wang, X.; Cui, X.; Xu, P.; Ji, C.; Guo, X.; You, L.; Gu, N.; Zeng, Y. (2018). Dynamic transcriptome profile in db/db skeletal muscle reveal critical roles for long noncoding RNA. Int. J. Biochem. Cell Biol. 104, 14-24.

## Claims

1. A nucleic acid molecule comprising
(i) the nucleotide sequence of SEQ ID NO: 1, 2, 3, 4 or 5 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii),
for use in medicine.

2. The nucleic acid molecule of claim 1 for use in medicine comprising
(i) the nucleotide sequence of SEQ ID NO: 1 or a functional fragment thereof,
(ii) a nucleotide sequence which has an identity of at least about 70%, at least about 80%, at least about 90% or at least about 99% to the nucleic acid molecule of (i), or
(iii) a nucleotide sequence which is complementary to the nucleotide sequence of the nucleic acid molecule of (i) or (ii)

3. The nucleic acid molecule of claim 1 or 2 for use in medicine, which is an RNA molecule, optionally comprising at least one modified building block.

4. The nucleic acid molecule of claim 1 or 2 for use in medicine, which is a DNA molecule, optionally in operative linkage with an expression control sequence and optionally present on a vector, e.g. a plasmid or a viral vector.

5. The nucleic acid molecule of any one of claims 1-4 for use in medicine, which is conjugated to a heterologous moiety.

6. A genome editing composition which is adapted for activating endogenous expression of a nucleic acid molecule of SEQ ID NO: 1, 2, 3, 4 or 5 or a nucleotide sequence which has an identity of at least about 70%, at least about 80%, at least about 90% or at least about 99% to the nucleotide sequence of SEQ ID NO: 1, 2, 3, 4 or 5 in a eukaryotic cell or organism, particularly a mammalian cell or organism, more particularly in a human cell or organism.

7. The genome editing composition of claim 6, which comprises
(i) a genome-editing enzyme such as a CRISPR/Cas enzyme, e.g. a CRISPR/Cas 9 or 13 enzyme, a transcription activator-like effector-based nuclease (TALEN), a zinc finger nuclease protein, a recombinase, a meganuclease, an Argonaute protein or
(ii) a nucleic acid molecule coding for a genome-editing enzyme.

8. A pharmaceutical preparation comprising a nucleic acid molecule of anyone of claims 1-5 or a genome editing composition of claim 6 or 7 and a pharmaceutically acceptable carrier.

9. The nucleic acid molecule of any one of claims 1-5, the genome editing composition of claim 6 or 7 or the pharmaceutical agent of claim 8 for use in human medicine.

10. The nucleic acid molecule of any one of claims 1-5, the genome editing composition of claim 6 or 7 or the pharmaceutical agent of claim 8 for use in the prevention or treatment of a cardiac disorder, particularly a cardiac hypertrophy-associated disorder.

11. The nucleic acid molecule of any one of claims 1-5, the genome editing composition of claim 6 or 7 or the pharmaceutical agent of claim 8 for use in the prevention or treatment of contractile dysfunction, cardiac decompensation or heart failure, e.g. diastolic heart failure, or atrial fibrillation, and/or for use in cardioprotection or cardioregeneration.

12. The nucleic acid molecule of any one of claims 1-5, the genome editing composition of claim 6 or 7 or the pharmaceutical agent of claim 8 for use in the prevention of treatment of a cardiac disorder wherein the compound is administered to patients selected from:
(i) patients having an increased risk for developing heart failure,
(ii) patients suffering from (congestive) heart failure, e.g. patients having an increased risk of heart failure progression;
(iii) post-myocardial infarction patients, and/or
(iv) patients with congenital heart diseases associated to cardiac hypertrophy, such as pulmonal vein stenosis, atrial or ventricular septum defects, and/or
(v) patients suffering from hypertrophic cardiomyopathy.

13. The nucleic acid molecule of any one of claims 1-5, the genome editing composition of claim 6 or 7 or the pharmaceutical preparation of claim 8 for use in the treatment or prevention of a cardiac disorder as monotherapy or in combination with a further medicament, particularly selected from angiotensin-modulating agents, β-blockers, diuretics, aldosterone antagonists, vasodilators, ionotropic agents, or combinations thereof, wherein the further medicament particularly is entresto.

14. A cell, organ or a non-human organism transfected or transformed with a nucleic acid molecule of anyone of claims 1-5 or a genome editing composition of claim 6 or 7.

15. A cell, organ or a non-human organism having an increased endogenous expression of a nucleic acid molecule of SEQ ID NO: 1, 2, 3, 4 or 5 or of a nucleotide sequence which has an identity of at least about 70%, at least about 80%, at least about 90% or at least about 99% to the nucleotide sequence of SEQ ID NO: 1, 2, 3, 4 or 5 compared to a wild-type cell, organ or organism.

16. A method of detecting, diagnosing or monitoring a cardiac disorder comprising detecting a nucleic acid molecule of claim 1 (i) or (ii) or of claim 2 (i) or (ii).

17. A reagent for detecting, diagnosing or monitoring a cardiac disorder comprising a reagent or reagent combination for detecting a nucleic acid molecule of claim 1 (i) or (ii) or of claim 2 (i) or (ii).
